# EUROPEAN PATENT SPECIFICATION

(11) **EP 2 950 813 B1**
(45) Date of publication and mention of the grant of the patent: **04.09.2019**
(21) Application number: 14704915.9
(22) Date of filing: 23.01.2014
(51) Int. Cl.: A61K 38/48, A61K 31/437, A61K 31/5377, A61P 7/04

(54) **COMPOSITIONS AND METHODS FOR COUNTERACTING FACTOR XA INHIBITION**
ZUSAMMENSETZUNG UND VERFAHREN ZUR ENTGEGENWIRKUNG GEGEN FAKTOR-XA-HEMMUNG
COMPOSITIONS ET PROCÉDÉS POUR CONTRER L'INHIBITION DU FACTEUR XA

(30) Priority: 31.01.2013 US 201361759332 P
(43) Date of publication of application: 09.12.2015
(73) Proprietor: Pfizer Inc., New York, NY 10017 (US); The Children's Hospital of Philadelphia, Philadelphia, PA 19104 (US)
(72) Inventor: CAMIRE, Rodney, Sicklerville, New Jersey 08081 (US); FRUEBIS, Joachim, Bedford, Massachusetts 01730 (US); PITTMAN, Debra D., Windham, New Hampshire 03087 (US)
(74) Representative: Pfizer
(86) International application number: PCT/IB2014/058494
(87) International publication number: WO 2014/118677

(56) References cited:
- WO-A1-2011/008885
- WO-A1-2013/049804
- WO-A2-2007/059513
- NABIL K THALJI: "Biochemistry and Cell Biology", ORAL PRESENTATIONS II, , 27 July 2013 (2013-07-27), page 16, XP009177338, Retrieved from the Internet: URL:http://www.ucdenver.edu/academics/coll eges/medicalschool/education/degree_progra ms/mstp/keystoneconference/Documents/2013_ MDPhD_Conference_Program.pdf [retrieved on 2014-04-01]
- Nabil K Thalji et al.: "Zymogen-Like FXa Is An Effective Pro-Hemostatic To Reverse The Anticoagulant Effects Of Direct FXa Inhibitors", , 8 December 2013 (2013-12-08), XP002722746, Retrieved from the Internet: URL:https://ash.confex.com/ash/2013/webpro gram/Paper63599.html [retrieved on 2014-04-01]
- "Combined Degree Retreat. August 1, 2012. Villanova University", Perelman School of Medicine , 1 August 2012 (2012-08-01), XP002722747, University of Pennsylvania Retrieved from the Internet: URL:https://www.med.upenn.edu/mstp/documen ts/RetreatAgenda2012.doc [retrieved on 2014-04-01]
- LACRAMIOARA IVANCIU ET AL: "A zymogen-like factor Xa variant corrects the coagulation defect in hemophilia", NATURE BIOTECHNOLOGY., vol. 29, no. 11, 23 October 2011 (2011-10-23), pages 1028-1033, XP055111224, US ISSN: 1087-0156, DOI: 10.1038/nbt.1995
- ELISABETH PERZBORN ET AL: "The discovery and development of rivaroxaban, an oral, direct factor Xa inhibitor", NATURE REVIEWS. DRUG DISCOVERY, vol. 10, no. 1, 1 January 2011 (2011-01-01), pages 61-75, XP055111223, GB ISSN: 1474-1776, DOI: 10.1038/nrd3185
- M. W. BUNCE ET AL: "Zymogen-like factor Xa variants restore thrombin generation and effectively bypass the intrinsic pathway in vitro", BLOOD, vol. 117, no. 1, 6 January 2011 (2011-01-06), pages 290-298, XP055111227, US ISSN: 0006-4971, DOI: 10.1182/blood-2010-08-300756
- PATRICIA QUADE-LYSSY, PETER MILANOV AND JÖRG SCHÜTTRUMPF: "Engineered Factor VII, Factor IX, and Factor X Variants for HemophiliaGene Therapy", J GENET SYNDR GENE THER, 2012, pages 1-7, XP002722748, ISSN: 2157-7412
- LU G ET AL: "Reconstructed recombinant factor Xa as an 5 antidote to reverse anticoagulation by factor Xa inhibitors", JOURNAL OF THROMBOSIS AND HAEMOSTASIS, vol. 7, no. suppl. 2, 1 July 2009 (2009-07-01), pages 309/OC-TH, XP009177312, BLACKWELL PUBLISHING, OXFORD, GB ISSN: 1538-7933
- HOLLENBACH S ET AL: "Bolus administration of PRT064445, a recombinant Factor Xa inhibitor antidote, reverses blood loss and PD markers in a rat model following enoxaparin induced anticoagulation", EUROPEAN HEART JOURNAL, vol. 33, no. Suppl, 1 January 2012 (2012-01-01), pages 309-310, XP009177324, OXFORD UNIVERSITY PRESS, GB ISSN: 0195-668X

### SEQUENCE LISTING

<110> PFIZER INC. THE CHILDREN'S HOSPITAL OF PHILADELPHIA
<120> COMPOSITIONS AND METHODS FOR COUNTERACTING FACTOR XA INHIBITION
<130> PC072006A
<150> US 61/759,332
   <151> 2013-01-31
<160> 3
<170> PatentIn version 3.5
<210> 1
   <211> 488
   <212> PRT
   <213> Homo sapiens
<400> 1
<210> 2
   <211> 1560
   <212> DNA
   <213> Homo sapiens
<400> 2
   gactttgctc cagcagcctg tcccagtgag gacagggaca cagtactcgg ccacaccatg 60
   gggcgcccac tgcacctcgt cctgctcagt gcctccctgg ctggcctcct gctgctcggg 120
   gaaagtctgt tcatccgcag ggagcaggcc aacaacatcc tggcgagggt cacgagggcc 180
   aattcctttc ttgaagagat gaagaaagga cacctcgaaa gagagtgcat ggaagagacc 240
   tgctcatacg aagaggcccg cgaggtcttt gaggacagcg acaagacgaa tgaattctgg 300
   aataaataca aagatggcga ccagtgtgag accagtcctt gccagaacca gggcaaatgt 360
   aaagacggcc tcggggaata cacctgcacc tgtttagaag gattcgaagg caaaaactgt 420
   gaattattca cacggaagct ctgcagcctg gacaacgggg actgtgacca gttctgccac 480
   gaggaacaga actctgtggt gtgctcctgc gcccgcgggt acaccctggc tgacaacggc 540
   aaggcctgca ttcccacagg gccctacccc tgtgggaaac agaccctgga acgcaggaag 600
   aggtcagtgg cccaggccac cagcagcagc ggggaggccc ctgacagcat cacatggaag 660
   ccatatgatg cagccgacct ggaccccacc gagaacccct tcgacctgct tgacttcaac 720
   cagacgcagc ctgagagggg cgacaacaac ctcaccagga tcgtgggagg ccaggaatgc 780
   aaggacgggg agtgtccctg gcaggccctg ctcatcaatg aggaaaacga gggtttctgt 840
   ggtggaacca ttctgagcga gttctacatc ctaacggcag cccactgtct ctaccaagcc 900
   aagagattca aggtgagggt aggggaccgg aacacggagc aggaggaggg cggtgaggcg 960
   gtgcacgagg tggaggtggt catcaagcac aaccggttca caaaggagac ctatgacttc 1020
   gacatcgccg tgctccggct caagaccccc atcaccttcc gcatgaacgt ggcgcctgcc 1080
   tgcctccccg agcgtgactg ggccgagtcc acgctgatga cgcagaagac ggggattgtg     1140
   agcggcttcg ggcgcaccca cgagaagggc cggcagtcca ccaggctcaa gatgctggag     1200
   gtgccctacg tggaccgcaa cagctgcaag ctgtccagca gcttcatcat cacccagaac     1260
   atgttctgtg ccggctacga caccaagcag gaggatgcct gccaggggga cagcgggggc     1320
   ccgcacgtca cccgcttcaa ggacacctac ttcgtgacag gcatcgtcag ctggggagag     1380
   ggctgtgccc gtaaggggaa gtacgggatc tacaccaagg tcaccgcctt cctcaagtgg     1440
   atcgacaggt ccatgaaaac caggggcttg cccaaggcca agagccatgc cccggaggtc     1500
   ataacgtcct ctccattaaa gtgagatccc actcaaaaaa aaaaaaaaaa aaaaaaaaaa     1560
<210> 3
   <211> 6
   <212> PRT
   <213> Artificial Sequence
<220>
   <223> Proteolytic Cleavage Site
<400> 3
   Arg Lys Arg Arg Lys Arg
   1               5

## Description

### CROSS-REFERENCE TO RELATED APPLICATION

This application claims the benefit of U.S. Provisional Application No. 61/759,332, filed 31 Jan 2013.

### REFERENCE TO SEQUENCE LISTING

The Sequence Listing is submitted concurrently herewith under 37 CFR §1.821 in a computer readable form (CRF) via EFS-Web as file name PC072006_SEQLIST_ST25.txt.

### BACKGROUND OF THE INVENTION

Pharmacological anticoagulation is the mainstay of treatment for patients with prothrombotic conditions. For over fifty years, the only oral anticoagulant available was warfarin, an inhibitor of the vitamin K epoxide reductase (VKOR) that recycles oxidized vitamin K. Warfarin has many drawbacks, including unpredictable pharmacokinetics that necessitate frequent monitoring of coagulation parameters and dose adjustment. However, in the event of emergency bleeding or the need for urgent surgery, antidotes exist that allow rapid and complete reversal.

Oral direct FXa inhibitors are emerging anticoagulants that have the potential to simplify dosing schemes and hemostatic monitoring in patients with prothrombotic diseases when compared to standard treatments, such as warfarin. Although these drugs have many advantages over warfarin, no fully efficacious reversal agent is available for these novel anticoagulants.

The lack of a specific countermeasure to their effects, however, is a critical unmet clinical need that could limit the widespread adoption of these agents due to fears of unmanageable bleeding.

### SUMMARY OF THE INVENTION

The invention is defined in the claims. Applicants have addressed this critical unmet clinical need by providing compositions and methods for counteracting the effects of direct activated Factor X (FXa) inhibitors. The invention is as set out in the claims in particular the present invention comprises; a Factor Xa variant comprising a substitution at the amino acid corresponding to position 235 in SEQ ID NO:1 selected from the group of amino acids consisting of: Thr or Leu, or a pharmaceutical composition thereof, for use in reducing or preventing bleeding in a subject being treated with a direct Factor Xa inhibitor; optionally wherein the use effects the urgent reversal of acquired coagulopathy due to FXa inhibition therapy in a subject with acute major bleeding.

According to some embodiments, the invention provides a Factor Xa variant for use in reducing or preventing bleeding in a subject being treated with a direct Factor Xa (FXa) inhibitor by administering a composition comprising a Factor Xa variant containing at least one modification including substitution for the wild-type amino acid at position 16 (using the chymotrypsin numbering system) with Thr, Leu, according to the disclosure Phe, Asp or Gly, or according to the disclosure substitution for the wild-type amino acid at position 17 (using the chymotrypsin numbering system) with Leu, Ala, or Gly. In certain embodiments, according to the invention the use of a composition comprising a FXa variant results in at least a 50% reduction in bleeding. According to certain embodiments, direct Factor Xa inhibitors include rivaroxaban or apixaban. In some embodiments according to the invention, the plasma concentration of the direct FXa inhibitor is a typical therapeutic amount or a supratherapeutic amount. For example, in some embodiments, the plasma concentration of rivaroxaban can be about 500 nM, or according to the disclosure greater, and the plasma concentration of apixaban can be about 250 nM, or according to the disclosure greater. According to certain embodiments of the invention the FXa variant contains the substitution I16L. In some embodiments, the FXa variant is capable of countering the effect of the direct Factor Xa inhibitor at a plasma concentration that is at least 100-fold lower than the plasma concentration of the Factor Xa inhibitor. In certain embodiments of the invention, the composition comprising the FXa variant is administered before a planned surgery, after an injury, or after an intentional or accidental overdose with a direct FXa inhibitor. In some disclosures, hemostasis in the subject is monitored using a hemostasis assay after a first dose with a FXa variant and, if adequate hemostasis is not attained by a predetermined time, at least one second dose of FXa variant is administered to achieve sufficient hemostasis. According to some embodiments, the predetermined time is about 15 mins, 30 mins, 45 mins or 60 mins after the first dose of FXa variant is administered. Other times are also possible. In some other embodiments of the invention, at least a second procoagulant is administered in addition to FXa variant, including for example, a different FXa variant, factor IX, factor Xla, factor XIIa, factor VIII, factor VIIa, FEIBA or prothrombin complex concentrate (PCC).

The disclosure provides methods for increasing the amount of thrombin produced in response to activation of the extrinsic or intrinsic clotting pathway in a subject being treated with a direct Factor Xa (FXa) inhibitor by administering a composition comprising a Factor Xa variant containing at least one modification including substitution for the wild-type amino acid at position 16 (using the chymotrypsin numbering system) with Thr, Leu, Phe, Asp or Gly, or substitution for the wild-type amino acid at position 17 (using the chymotrypsin numbering system) with Leu, Ala, or Gly. According to certain disclosures, direct Factor Xa inhibitors include rivaroxaban or apixaban. In some disclosures, the plasma concentration of the direct FXa inhibitor is a typical therapeutic amount or a supratherapeutic amount. For example, in some disclosures, the plasma concentration of rivaroxaban can be about 500 nM, or greater, and the plasma concentration of apixaban can be about 250 nM, or greater. According to certain embodiments the FXa variant contains the substitution I16L. According to certain disclosures, the amount of thrombin produced increases by about 5%, 10%, 15%, 20%, 30%, 40%, 50%, 60%, 70%, 80%, 90%, 100%, 150%, 200%, or more. In some disclosures, the FXa variant is capable of countering the effect of the direct Factor Xa inhibitor at a plasma concentration that is at least 100-fold lower than the plasma concentration of the Factor Xa inhibitor. In certain disclosures, the composition comprising the FXa variant is administered before a planned surgery, after an injury, or after an intentional or accidental overdose with a direct FXa inhibitor. In some disclosures, hemostasis in the subject is monitored using a hemostasis assay after a first dose with a FXa variant and, if adequate hemostasis is not attained by a predetermined time, at least one second dose of FXa variant is administered to achieve sufficient hemostasis. According to some disclosures, the predetermined time is about 15 mins, 30 mins, 45 mins or 60 mins after the first dose of FXa variant is administered. Other times are also possible. In some other disclosures, at least a second procoagulant is administered in addition to FXa variant, including for example, a different FXa variant, factor IX, factor Xla, factor XIIa, factor VIII, factor VIIa, FEIBA or prothrombin complex concentrate (PCC).

According to some disclosures, the disclosure provides methods for decreasing clotting time (as measured, for example, using PT or INR, or some other assay) in a subject being treated with a direct Factor Xa (FXa) inhibitor by administering a composition comprising a Factor Xa variant containing at least one modification including substitution for the wild-type amino acid at position 16 (using the chymotrypsin numbering system) with Thr, Leu, Phe, Asp or Gly, or substitution for the wild-type amino acid at position 17 (using the chymotrypsin numbering system) with Leu, Ala, or Gly. According to certain disclosures, direct Factor Xa inhibitors include rivaroxaban or apixaban. In some disclosures, the plasma concentration of the direct FXa inhibitor is a typical therapeutic amount or a supratherapeutic amount. For example, in some disclosures, the plasma concentration of rivaroxaban can be about 500 nM, or greater, and the plasma concentration of apixaban can be about 250 nM, or greater. According to certain disclosures the FXa variant contains the substitution I16L. According to certain disclosures, clotting time is reduced by about 5%, 10%, 15%, 20%, 30%, 40%, 50%, 60%, 70%, 80%, 90%, or more. In some disclosures, the FXa variant is capable of countering the effect of the direct Factor Xa inhibitor at a plasma concentration that is at least 100-fold lower than the plasma concentration of the Factor Xa inhibitor. In certain disclosures, the composition comprising the FXa variant is administered before a planned surgery, after an injury, or after an intentional or accidental overdose with a direct FXa inhibitor. In some disclosures, hemostasis in the subject is monitored using a hemostasis assay after a first dose with a FXa variant and, if adequate hemostasis is not attained by a predetermined time, at least one second dose of FXa variant is administered to achieve sufficient hemostasis. According to some disclosures, the predetermined time is about 15 mins, 30 mins, 45 mins or 60 mins after the first dose of FXa variant is administered. Other times are also possible. In some other disclosures, at least a second procoagulant is administered in addition to FXa variant, including for example, a different FXa variant, factor IX, factor Xla, factor XIIa, factor VIII, factor VIIa, FEIBA or prothrombin complex concentrate (PCC).

### BRIEF DESCRIPTION OF THE DRAWINGS

Figures 1A-B show inhibition of free wt-FXa or FXa^{I16L} by rivaroxaban. The initial velocity of peptidyl substrate (SpecXa; 200 uM) hydrolysis by A) wt-FXa (2 nM) or B) FXa^{I16L} (6 nM) was determined at increasing concentrations of rivaroxaban. The Ki value is given on each graph.
Figures 2A-B show rivaroxaban inhibition of wt-FXa or FXa^{I16L} assembled in prothrombinase. The initial velocity of peptidyl substrate (SpecXa; 200 uM) hydrolysis by A) wt-FXa (2 nM) or B) FXa^{I16L} (6 nM) in the presence of PCPS (20 uM) and FVa (40 nM) was determined at increasing concentrations of rivaroxaban.
Figure 3 shows the effect of different concentrations of FXa^{I16L} on reversing the effects on thrombin generation of rivaroxaban.
Figures 4A-D show the effect of FXa^{I16L} on reversing the effects of rivaroxaban. Normal human plasma was incubated with 500 nM rivaroxaban and in the absence or presence of increasing concentrations of FXa^{I16L}. Following data analysis, peak thrombin (A and C) and total thrombin generated (ETP; B and D) were plotted.
Figures 5A-B show FXa^{I16L} reverses the effects of high dose rivaroxaban. Normal human plasma was incubated with 7.5 uM rivaroxaban and in the absence or presence of increasing concentrations of FXa^{I16L}. Following data analysis, peak thrombin (A) and total thrombin generated (ETP; B) were plotted.
Figures 6A-B show FXa^{I16L} or FXa^{I16T} reverses the effects of 250 nM apixaban. Normal human plasma was incubated with 250 nM apixaban and in the absence or presence of increasing concentrations of FXa^{I16L} or FXa^{I16T}. Following data analysis, peak thrombin (A) and total thrombin generated (ETP; B) were plotted.
Figures 7A-B show FXa^{I16L} or FXa^{I16T} reverses the effects of high dose apixaban. Normal human plasma was incubated with 2.0 uM Apixaban and in the absence or presence of increasing concentrations of FXa^{I16L} or FXa^{I16T}. Following data analysis, peak thrombin (A) and total thrombin generated (ETP; B) were plotted.
Figures 8A-B show FXa^{I16L} corrects whole blood clotting in the presence of rivaroxaban. Whole blood thromboelastography was used to assess the ability of FXa^{I16L} to reverse the effects of rivaroxaban at a typical (A) and a high (B) dose.
Figures 9A-B show FXa^{I16L} corrects whole blood clotting in the presence of apixaban. Whole blood thromboelastography was used to assess the ability of FXa^{I16L} to reverse the effects of apixaban at a typical (A) and a high (B) dose.
Figures 10A-B show that FXa^{I16L} counteracts rivaroxaban in a thrombin generation assay. Figure 10A shows a dose response of rivaroxaban and Figure 10B shows a dose response of FXa^{I16L} in the presence of rivaroxaban.
Figure 11 shows that FXa^{I16L} counteracts rivaroxaban in a mouse tail clip bleeding model.
Figure 12 demonstrates that rivaroxaban administered to mice delays clotting time of whole blood measured using ROTEM and that administration with FXa^{I16L} dose-responsively counteracts the effect of rivaroxaban.

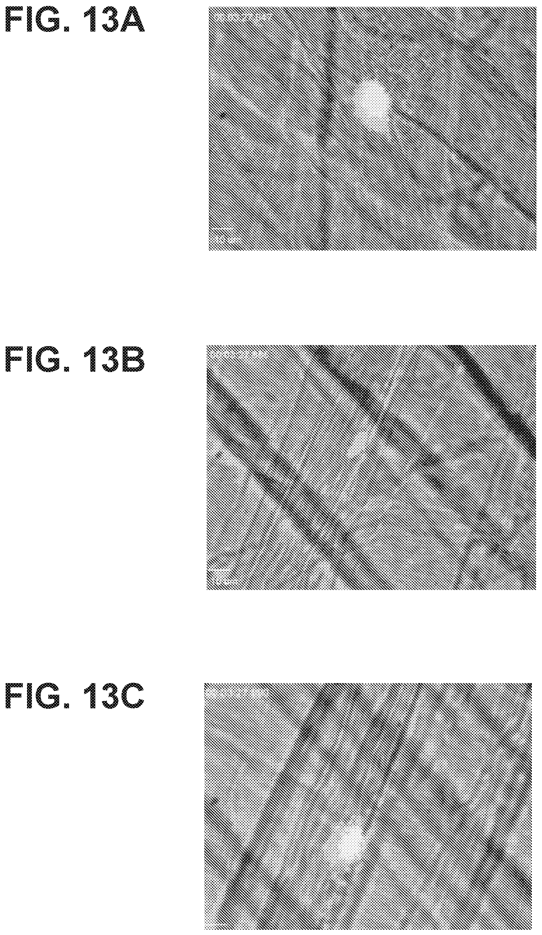

Figure 13 shows that rivaroxaban administered to a mouse prevents clot formation at a site of vascular injury in the cremaster muscle caused by laser and that administration with FXa^{I16L} counteracts the effect of rivaroxaban. Clot formation was visualized using intravital microscopy and fluorescently labeled antibodies against fibrin and platelets. Figure 13A shows clot formation in an untreated mouse. Figure 13B shows that rivaroxaban delayed and reduced platelet accumulation and prevented fibrin deposition. By contrast, Figure 13C shows that in a mouse administered rivaroxaban and FXa^{I16L} clot formation occurred at the site of injury.

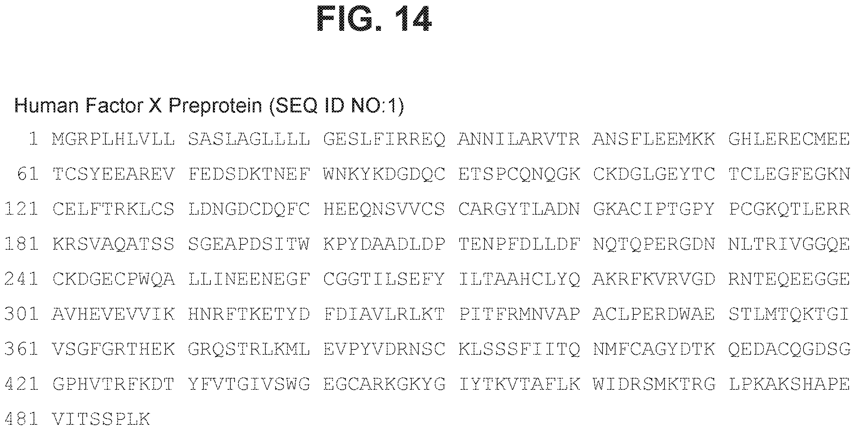

Figure 14 is the amino acid sequence of wild-type human Factor X preprotein (SEQ ID NO:1). The signal peptide corresponds to amino acids 1-23. The propeptide corresponds to amino acids 24-40. The mature light chain of activated Factor X (FXa) corresponds to amino acids 41-179. The mature heavy chain of activated FXa (after removal of the activation peptide) corresponds to amino acids 235-488. The activation peptide (AP) corresponds to amino acids 183-234.

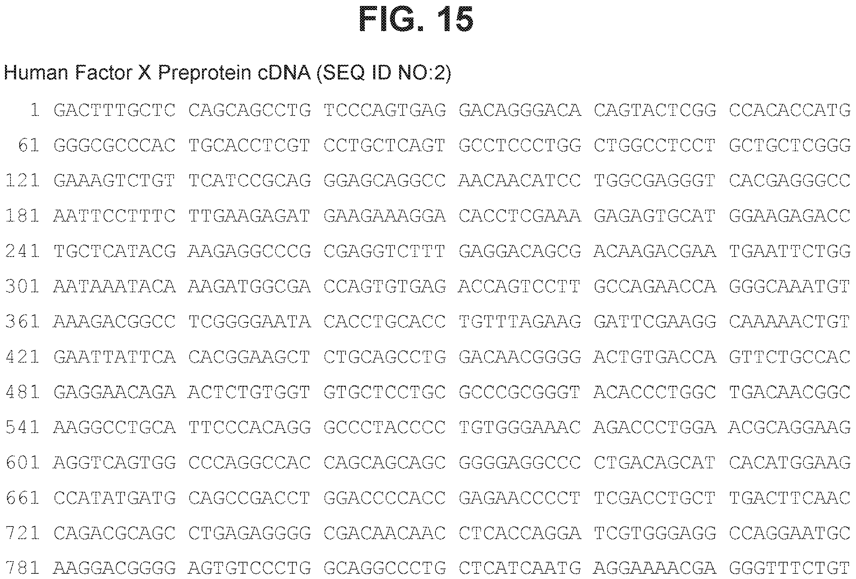

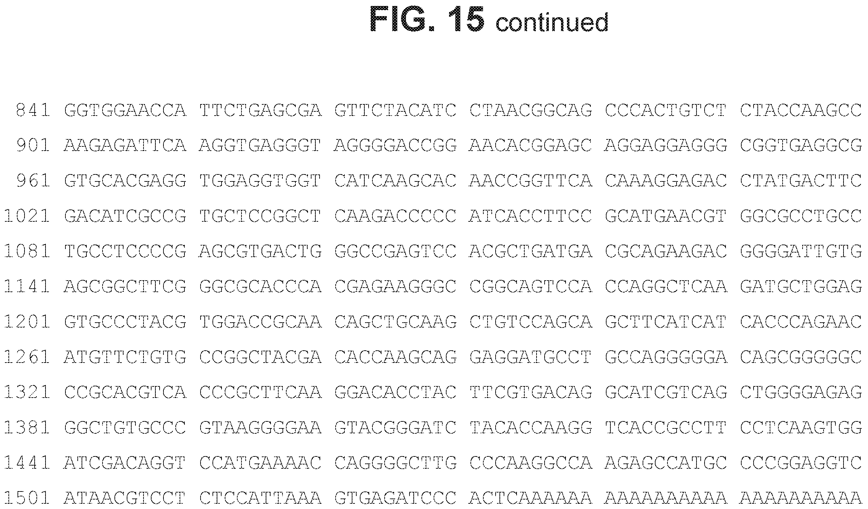

Figure 15 is the nucleotide sequence of the cDNA encoding wild-type human Factor X preprotein (SEQ ID NO:2). The coding sequence corresponds to nucleotides 58 to 1524.

### DETAILED DESCRIPTION

The disclosure provides compositions and methods for counteracting the anti-coagulant effect of a direct FXa inhibitor in a subject in need thereof. Applicants have discovered that certain FXa variants rapidly and completely counteract the effect of a direct FXa inhibitor in a dose dependent manner. More specifically, applicants have discovered that a relatively small amount of an FXa variant restores normal coagulation activity *in vivo* in the presence of FXa inhibitor at therapeutic concentrations and even at supratherapeutic concentrations. By providing fast and effective antidotes for the anti-coagulation effects of direct FXa inhibitors, Applicants' disclosure therefore contributes to fulfilling the promise of these advantageous anti-coagulants.

Coagulation factor X (FX) is a zymogen which, upon activation by the intrinsic factor IX/factor VIII or extrinsic pathway (tissue factor/factor VIIa), becomes FXa, which is the protease moiety of prothrombinase. Following proteolytic cleavage of the Arg-Ile scissile bond, releasing an activation peptide (AP), a series of well defined structural changes in the zymogen drives the activation process to the mature active serine protease (See Toso et al., (2008) J. Biol. Chem. 283, 18627-18635; Bunce et al., (2011) Blood 117, 290-298; and Ivanciu et al., (2011) Nat. Biotechnol. 29, 1028-1033, incorporated by reference herein in their entirety). The mature FXa has a light chain and a heavy chain that contains the catalytic domain. The mature FXa becomes an active serine protease upon formation of the prothrombinase complex, which includes binding of an activated cofactor, Factor Va (FVa).

Variant forms of FX have been developed that upon activation cleavage yield a zymogen-like FXa variant. That is, once cleaved, the resulting FXa variant has poor active site function and is more resistant to inactivation by circulating inhibitors (i.e. antithrombin III and TFPI). The FXa variants, thus, have longer half-lives in plasma than wild-type FXa. The FXa variant binds FVa, lipid membrane and calcium to form a fully active prothrombinase complex that efficiently activates prothrombin.

The zymogen-like variants of FXa circulate in the zymogen-like conformation and do not seem to be thrombogenic (See Toso et al., (2008) J. Biol. Chem. 283, 18627-18635 and Ivanciu et al., (2011) Nat. Biotechnol. 29, 1028-1033, incorporated by reference herein in their entirety). Examples of such FXa variants are described in International patent publication WO2007/059513, incorporated herein by reference in its entirety.

The enzymes of coagulation are trypsin-like enzymes that belong to the S1 peptidase family of proteases that bear a chymotrypsin-like fold. The coagulation proteases contain catalytic domains that are highly homologous to each other and to the ancestral serine proteases of digestion. The structural homology/identity is so great (>70%) that residues in the catalytic domains of the coagulation enzymes (including Factor Xa) are numbered according to the corresponding residues in chymotrypsinogen. (Chymotrypsin numbering system; see Bajaj and Birktoft, Methods Enzymol. 1993; 222:96-128, Table 2, and Bode W, Mayr I, Bauman Y, et al. The refined 1.9 Å crystal structure of human alpha-thrombin: interaction with D-Phe-Pro-Arg chloromethylketone and significance of the Tyr-Pro-Trp insertion segment. EMBO J 1989;8(11):3467-3475, both of which are incorporated by reference herein in their entireties). Accordingly, amino acids may be referred to herein according to the chymotrypsin numbering system, which is well-known to those of skill in the art.

According to the disclosure, an FXa variant may be an FXa protein comprising an amino acid substitution that makes the variant more zymogen-like compared to a wild-type FXa protein *in vivo* or *in vitro.* FXa variants of the disclosure substantially regain wild-type FXa activity upon formation of prothrombinase. Examples of FXa variants that are useful in the uses of the invention are variants comprising a modification selected from the group consisting of: a) Ile at position 16 is Thr, Leu, according to the disclosure Phe, Asp or Gly and b) Val at position 17 is Leu, Ala, or Gly, according to the chymotrypsin numbering system. Amino acids 16 and 17 in the chymotrypsin numbering system occur at amino acids 235 and 236, respectively, of SEQ ID NO:1 (human Factor X preproprotein). In certain embodiments of the invention, FXa variants are FXa^{I16L} and FXa^{I16T} (the nomenclature used herein for the FXa variants recites the original amino acid at the numbered position according to the chymotrypsin numbering system followed by the substituted amino acid). The FXa variants can be variants of any mammalian FXa. Of particular interest, however, are FXa variants of human FXa.

In certain disclosures, the FX variant that is activated into a variant FXa of the disclosure may be further modified by inserting a non-native intracellular proteolytic cleavage site. In a non-limiting example, to express "activated" zymogen-like FXa variants in mammalian cells, a non-native intracellular proteolytic cleavage site can be inserted between the Arg at position 234 of SEQ ID NO:1 (position 15 in the chymotrypsin numbering system) and the amino acid at the position corresponding to position 235 of SEQ ID NO:1 (position 16 in the chymotrypsin numbering system) in the variant FX zymogen. In certain disclosures, the non-native intracellular protease cleavage site is Arg-Lys-Arg or Arg-Lys-Arg-Arg-Lys-Arg (SEQ ID NO:3). These cleavage sites are efficiently recognized by proteases (PACE/furin-like enzymes) within the cell and are removed. This cleavage may result in a processed variant FXa in which the mature heavy chain of the molecule now begins at the amino acid at the position corresponding to position 235 of SEQ ID NO:1 (position 16 in the chymotrypsin numbering system). Introduction of this cleavage site at said position allows for the intracellular conversion of FX to FXa.

In certain disclosures the entire amino acid sequence of the FX variant activation peptide (AP) (i.e., amino acids 183-234 of SEQ ID NO:1) is replaced with a non-native intracellular protease cleavage site. According to certain disclosures, the non-native intracellular protease cleavage site is Arg-Lys-Arg or Arg-Lys-Arg-Arg-Lys-Arg (SEQ ID NO:3). As explained above, this modification allows for intracellular cleavage of the FX variant expressed by cells. The intracellular cleavage converts FX variant to activated zymogen-like FXa variant which is then secreted by cells for subsequent purification. This approach obviates the need for extracellular cleavage that would otherwise be required to activate the variant clotting factor, for example, after isolating the protein or just before blood clotting.

In certain disclosures, FXa variants of the disclosure are derived from FX variant preproteins comprising native wild-type human signal sequence and/or propeptide sequence. In other disclosures, FX signal sequences and/or propeptide from non-human species can be used in place of the corresponding native amino acid sequences. And in yet other disclosures, signal sequence and/or propeptide sequence from other human or non-human secreted proteins can be used in place of the corresponding native amino acid sequences.

In an exemplary embodiment of the invention, a FXa variant comprises amino acids 41-179 and amino acids 235-488 of SEQ ID NO:1, wherein the amino acid at position 235 (isoleucine in the wild-type sequence) is substituted with a different amino acid selected from the group consisting of according to the invention threonine (Thr), leucine (Leu), according to the disclosure phenylalanine (Phe), aspartic acid (Asp), or glycine (Gly). These substitutions can respectively be written using the nomenclature I235T, I235L, I235F, I235D and I235G, where the first letter is the single letter code for isoleucine and the last letter is the single letter code for the amino acid being substituted into the wild-type sequence. Because position 235 of SEQ ID NO:1 is equivalent to position 16 in the chymotrypsin numbering system, the same substitutions can be written I16T, I16L, I16F, I16D and I16G. In an embodiment of the invention, a FXa variant comprises amino acids 41-179 and amino acids 235-488 of SEQ ID NO:1, wherein the amino acid at position 235 is substituted with Thr (i.e., I235T or I16T). In an embodiment of the invention, a FXa variant comprises amino acids 41-179 and amino acids 235-488 of SEQ ID NO:1, wherein the amino acid at position 235 is substituted with Leu (i.e., I235L or I16L). In a disclosure, a FXa variant comprises amino acids 41-179 and amino acids 235-488 of SEQ ID NO:1, wherein the amino acid at position 235 is substituted with Phe (i.e., I235F or I16F). In a disclosure, a FXa variant comprises amino acids 41-179 and amino acids 235-488 of SEQ ID NO:1, wherein the amino acid at position 235 is substituted with Asp (i.e., I235D or I16D). In a disclosure, a FXa variant comprises amino acids 41-179 and amino acids 235-488 of SEQ ID NO:1, wherein the amino acid at position 235 is substituted with Gly (i.e., I235G or I16G).

According to another exemplary disclosure, a FXa variant comprises amino acids 41-179 and amino acids 235-488 of SEQ ID NO:1, wherein the amino acid at position 236 (valine in the wild-type sequence) is substituted with a different amino acid selected from the group consisting of leucine (Leu), alanine (Ala), or glycine (Gly). These substitutions can respectively be written using the nomenclature V236L, V236A, and V236G, where the first letter is the single letter code for valine and the last letter is the single letter code for the amino acid being substituted into the wild-type sequence. Because position 236 of SEQ ID NO:1 is equivalent to position 17 in the chymotrypsin numbering system, the same substitutions can be written V17L, V17A, and V17G. In a disclosure, a FXa variant comprises amino acids 41-179 and amino acids 235-488 of SEQ ID NO:1, wherein the amino acid at position 236 is substituted with Leu (i.e., V236L or V17L). In a disclosure, a FXa variant comprises amino acids 41-179 and amino acids 235-488 of SEQ ID NO:1, wherein the amino acid at position 236 is substituted with Ala (i.e., V236A or V17A). In a disclosure, a FXa variant comprises amino acids 41-179 and amino acids 235-488 of SEQ ID NO:1, wherein the amino acid at position 236 is substituted with Gly (i.e., V236G or V17G).

In other disclosures, FXa variants of the disclosure, including those specific variants described in the preceding paragraphs, can include various isoforms of the light and/or mature heavy chain of the protein. Non-limiting exemplary isoforms of the FXa variant mature heavy chain include the alpha and beta versions of the mature heavy chain. Jesty et al., J Biol Chem. 1975 Jun 25;250(12):4497-504, incorporated by reference herein. Compositions of the disclosure can include FXa variant proteins in which one or the other or both alpha and beta mature heavy chain isoforms are represented.

According to yet other disclosures, isoforms of FXa variant proteins, including those specific variants described in the preceding paragraphs, can include isoforms in which a variable number of amino acids (for example, 1, 2, 3, 4, 5, 6, or more amino acids) are either missing from or added to the carboxy terminus of the light chain and/or mature heavy chains of the protein.

According to certain disclosures, FXa variants of the disclosure include proteins with a certain minimal degree of homology or sequence identity compared to the amino acid sequence of wild-type FXa in SEQ ID NO:1. Thus, for example, FXa variants include proteins that contain a light and mature heavy chain that are at least 60%, 70%, 80%, 85%, 90%, 95%, 98%, or 99% homologous or identical in sequence with the wild-type FXa light and mature heavy chains in SEQ ID NO:1, wherein such FXa variants also include a substitution at the amino acid position corresponding to position 235 of SEQ ID NO:1 with Thr, Leu, Phe, Asp, or Gly, or a substitution at the amino acid position corresponding to position 236 of SEQ ID NO:1 with Leu, Ala, or Gly, and further wherein such FXa variants are zymogenic until incorporated into prothrombinase complex. In the amino acid sequence of SEQ ID NO:1, the wild-type FXa light chain sequence corresponds to amino acids 41 to 179 and the wild-type FXa mature heavy chain sequence corresponds to amino acids 235 to 488. Percentage amino acid sequence homology or identity can readily be determined using software such as Protein BLAST available at the website of the National Center for Biotechnology Information (http://blast.ncbi.nlm.nih.gov/Blast.cgi).

According to other non-limiting disclosures, FXa variants of the disclosure can also include FXa variants containing one or more post-translational modifications including, without limitation, one or more O-linked or N-linked carbohydrate groups or a variable number of gamma-carboxyglutamic acid (Gla) residues. FXa variants of the disclosure can further include chemically modified FXa variant proteins. Other FXa variants useful in the methods of the disclosure are also possible.

As used herein, the term FXa^{I16x} refers to a variant of activated Factor X wherein the amino acid corresponding to position 235 in SEQ ID NO:1 (corresponding to position 16 in the chymotrypsin numbering system) is changed from the amino acid in the wild-type sequence (isoleucine) to a different amino acid denoted "x". In some non-limiting exemplary disclosures, amino acid "x" can be threonine (Thr or T), leucine (Leu or L), phenylalanine (Phe or F), aspartic acid (Asp or D), or glycine (Gly or G).

As used herein, the term FXa^{V17y} refers to a variant of activated Factor X wherein the amino acid corresponding to position 236 in SEQ ID NO:1 (corresponding to position 17 in the chymotrypsin numbering system) is changed from the amino acid in the wild-type sequence (valine) to a different amino acid denoted "y". In some non-limiting exemplary disclosures, amino acid "y" can be leucine (Leu or L), alanine (Ala or A), or glycine (Gly or G).

The terms FXa^{I16x} and FXa^{V17y} are not limited by the protein sequence set forth in SEQ ID NO:1. Rather these terms additionally include the variety of isoforms and homologous proteins described herein with the specified substitution mutations at positions 16 or 17 in the chymotrypsin numbering system that behave as zymogens until incorporated into prothrombinase complex.

An FXa variant of the disclosure may be produced by any technique for expressing a protein.

An "isolated protein," "isolated polypeptide" or "isolated variant" is a protein, polypeptide or variant that by virtue of its origin or source of derivation (1) is not associated with naturally associated components that accompany it in its native state, (2) is free of other proteins from the same species, (3) is expressed by a cell from a different species, or (4) does not occur in nature. Thus, a polypeptide that is chemically synthesized or synthesized in a cellular system different from the cell from which it naturally originates will be "isolated" from its naturally associated components. A protein may also be rendered substantially free of naturally-associated components by isolation, using protein purification techniques well known in the art.

A protein or polypeptide is "substantially pure," "substantially homogeneous," or "substantially purified" when at least about 60 to 75% of a sample exhibits a single species of polypeptide. The polypeptide or protein may be monomeric or multimeric. A substantially pure polypeptide or protein will typically comprise about 50%, 60%, 70%, 80% or 90% W/W of a protein sample, more usually about 95%, and may be over 99% pure. Protein purity or homogeneity may be indicated by a number of means well known in the art, such as polyacrylamide gel electrophoresis of a protein sample, followed by visualizing a single polypeptide band upon staining the gel with a stain well known in the art. For certain purposes, higher resolution may be provided by using HPLC or other means well known in the art for purification.

The methods of the disclosure are useful to counteract a direct FXa inhibitor. A direct FXa inhibitor is an inhibitor that binds directly to FXa and selectively binds FXa over other proteases. Direct FXa inhibitors are noncompetitive inhibitors of FXa with respect to prothrombin. They bind the substrate binding cleft and inhibit FXa competitively with respect to small peptide substrates that also bind this region. They inhibit FXa with high picomolar affinity and are highly protein bound in plasma. Examples of direct FXa inhibitors are rivaroxaban, apixaban, betrixaban, darexaban, edoxaban and otamixaban. In certain disclosures, direct FXa inhibitors are selected from rivaroxaban and apixaban.

According to the disclosure, an FXa variant can be used to counteract a direct FXa inhibitor that binds FXa or that binds FXa that has formed prothrombinase. The direct FXa inhibitors may or may not require cofactors of FXa for inhibition. According to the methods of the disclosure, an FXa variant, such as FXa^{I16L} and FXa^{I16T}, are administered to a subject whose blood contains a direct FXa inhibitor.

The disclosure encompasses the use of a FXa variant to counteract direct FXa inhibitors, including but not limited to synthetic inhibitors, small molecule inhibitors, orally available inhibitors, or reversible inhibitors. The FXa inhibitor may be any combination of these features, such as an orally available, synthetic, reversible, small molecule inhibitor. In certain embodiments of the invention, the direct FXa inhibitors may be selected from rivaroxaban, apixaban, or as disclosed, betrixaban, darexaban, edoxaban and otamixaban (see Perzborn et al., Nat Rev Drug Discov. 2011 Jan;10(1):61-75; Turpie, Arterioscler Thromb Vasc Biol. 2007 Jun;27(6):1238-47; Pinto et al., Expert Opin. Ther. Patents 22:645-661 (2012); Pinto, et al., J. Med. Chem. 50:5339-5356 (2007)). In certain embodiments of the invention, direct FXa inhibitors are selected from rivaroxaban or apixaban.

In some embodiments, a FXa variant of the disclosure can be administered to a subject to reverse the effects of a direct FXa inhibitor where such inhibitor occurs at therapeutic concentrations. In other embodiments of the invention, a FXa variant of the according to the invention can be administered to a subject to reverse the effects of a direct FXa inhibitor where such inhibitor occurs at supratherapeutic concentrations. A supratherapeutic concentration is one that is higher than that ordinarily considered required to safely achieve anti-coagulation in a particular subject or class of subjects. Supratherapeutic concentrations of a direct FXa inhibitor can result from accidental or intentional overdose. Supratherapeutic concentrations of a direct FXa inhibitor can also result from unexpected effects in particular subjects, such as an unexpectedly high sensitivity to these drugs, or unexpectedly slow rate of clearance, due for example to drug interactions or other factors. Determination of what would be a therapeutic concentration or supratherapeutic concentration of direct FXa inhibitor in a particular subject or class of subjects is within the knowledge of those ordinarily skilled in the art.

According to the disclosure, an FXa variant is used to counteract a direct FXa inhibitor or inhibitors that selectively bind FXa over other trypsin-like proteases by at least 5-fold, at least 6-fold, at least 7-fold, at least 10-fold, at least 15-fold, at least 20-fold, at least 25-fold, at least 30-fold, at least 50-fold, at least 100-fold, at least, 500-fold, at least 1,000-fold, at least 5,000-fold or at least 10,000-fold.

The direct FXa inhibitor may bind an FXa variant with a Kᵢ of about 2 x 10⁻⁷ M or less. "Kᵢ" refers to the inhibitor constant of a particular inhibitor-target interaction, which is the concentration required to produce half maximum inhibition. One can determine the Kᵢ by using methods known in the art. The disclosure contemplates, thus, counteracting a direct FXa inhibitor that binds an FXa variant free of the prothrombinase complex with a Kᵢ of about 2 x 10⁻⁸ M or less, about 1 x 10⁻⁸ M or less, about 9 x 10⁻⁹ M or less, about 8 x 10⁻⁹ M or less, about 7 x 10⁻⁹ M or less, about 6 x 10⁻⁹ M or less, about 5 x 10⁻⁹ M or less, about 4 x 10⁻⁹ M or less, about 3 x 10⁻⁹ M or less, about 2 x 10⁻⁹ M or less, about 1 x 10⁻⁹ M or less, about 9 x 10⁻¹⁰ M or less, about 8 x 10⁻¹⁰ M or less, about 7 x 10⁻¹⁰ M or less, about 6 x 10⁻¹⁰ M or less, about 5 x 10⁻¹⁰ M or less, about 4 x 10⁻¹⁰ M or less, about 3 x 10⁻¹⁰ M or less, about 2 x 10⁻¹⁰ M or less, about 1 x 10⁻¹⁰ M or less, about 9 x 10⁻¹¹ M or less, about 8 x 10⁻¹¹ M or less, about 7 x 10⁻¹¹ M or less, about 6 x 10⁻¹¹ M or less, about 5 x 10⁻¹¹ M or less, about 4 x 10⁻¹¹ M or less, about 3 x 10⁻¹¹ M or less, about 2 x 10⁻¹¹ M or less, about 1 x 10⁻¹¹ M or less, about 9 x 10⁻¹² M or less, about 8 x 10⁻¹² M or less, about 7 x 10⁻¹² M or less, about 6 x 10⁻¹² M or less, about 5 x 10⁻¹² M or less, about 4 x 10⁻¹² M or less, about 3 x 10⁻¹² M or less, about 2 x 10⁻¹² M or less, or about 1 x 10⁻¹² M or less, or less. The direct FXa inhibitor to be counteracted by an FXa variant according to the methods of the disclosure may bind a wild-type FXa with a Kᵢ at least 1.5 fold, at least 2-fold, at least 3-fold, at least 4-fold, at least 5-fold, at least 6-fold, at least 7-fold, at least 10-fold, at least 15-fold, at least 20-fold, at least 25-fold, at least 30-fold, or at least 50-fold less than it binds the FXa variant. The direct FXa inhibitor may bind a wild-type FXa with a Kᵢ of at least 20%, at least 30%, at least 40%, at least 50%, at least 60%, at least 70%, at least 80%, at least 90%, at least 95%, or at least 99% less than the Kᵢ with an FXa variant free of the prothrombinase complex. The direct FXa inhibitor may bind a prothrombinase complex comprising a wild-type FXa with about the same Kᵢ as it binds a prothrombinase complex comprising an FXa variant.

In one aspect, the disclosure provides methods for counteracting the effects of a direct FXa inhibitor in a subject who is bleeding (internally or externally) or is at risk of bleeding (e.g., in the course of a planned surgery) by administering a FXa variant. In some disclosures, the direct FXa inhibitor may be present in the subject at a therapeutic concentration or a higher concentrations (i.e., a supratherapeutic concentration). In some disclosures, the therapeutic concentration may be an overdose in sensitive individuals. The methods of the disclosure, thus, are useful for providing an antidote to an overdose of a direct FXa inhibitor. In various disclosures, the subject of treatment may be a human or a veterinary subject.

Direct inhibitor overdose can be detected based on existence of symptoms or signs of excessively reduced clotting ability. Non-limiting examples include evidence of gastrointestinal bleeding, including dark tarry stools, bloody stools, and vomiting of blood. Other examples include nosebleeds, and increased tendency to, or severity of, bruising or bleeding from minor cuts and scrapes.

In a clinical setting, direct inhibitor overdose can be detected directly or by measuring the ability of subject blood to clot and detecting deviations from the expected degree of anti-coagulation. Blood clotting potential can be measured in ways familiar to those ordinarily skilled in the art. For example, overdose may be suspected when a subject's prothrombin time is excessively prolonged. In certain embodiments, overdose is confirmed when the prothrombin time expressed as an International Normalized Ratio (INR) is measured to be greater than about 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10, 12, 14, 16, 18, 20, or greater.

The FXa variant may be administered whenever it is desired to counteract the effects of the direct FXa inhibitor, including but not limited to before a planned surgery, after an injury resulting in external or internal bleeding or after a direct FXa inhibitor overdose. According to the disclosure, the FXa variant may be administered at least about 12 hours, at least about 6 hours, at least about 3 hours, at least about 2 hours, at least about 1 hour, at least about 30 minutes, at least about 10 minutes, or at least about 5 minutes of when the desired counteracting effect is needed, such as before a planned surgery, after an injury resulting in external or internal bleeding or after a direct FXa inhibitor overdose.

According to another embodiment, the invention provides a FXa variant for use according to the invention to effect the urgent reversal of acquired coagulopathy due to FXa inhibition therapy in a subject with acute major bleeding. In some disclosures, subjects are adult human patients. In other disclosures, subjects are pediatric human patients.

In some embodiments, acute major bleeding is caused by trauma. In other embodiments, acute major bleeding occurs during surgery or as disclosed other type of interventional procedure. Exemplary disclosed non-limiting interventional procedures include incisions, drainage, vascular surgery, appendectomy, herniotomy or hernioplasty, abdominal surgery, cholecystectomy, trephination (burr hole), lumbar puncture, cardiac pacemaker insertion, hip fracture surgery, and others. In yet other disclosures, acute major bleeding can be spontaneous bleeding with no apparent cause.

Without limitation, sites of acute major bleeding include gastrointestinal bleeding, subcutaneous or intramuscular bleeding, bladder bleeding, hemarthrosis, subdural hematoma, nasal bleeding, peritoneal bleeding, uterine bleeding, and other sites of bleeding.

Effective treatment with FXa variants of the disclosure can reverse the effects of a direct FXa inhibitor. Successful reversal of such effects by a FXa variant can be determined in a variety of ways and be measured or monitored using different assays, methods, or endpoints.

In some disclosures, treatment with a FXa variant to reverse the effects of a direct FXa inhibitor is monitored using tests or assays performed on blood or plasma from a subject treated with FXa variant. A blood sample can be taken from a subject at a predetermined time after treatment with FXa variant. The blood, or plasma prepared from it, is then subjected to one or more tests to determine if certain hemostatic pharmacodynamic parameters have been normalized despite the presence of direct FXa inhibitor. If normalization is found then the subject need not be further treated with FXa variant. If normalization is not found, however, then further treatment with FXa variant in accordance with the methods of the disclosure may be required to reverse the effect of a direct FXa inhibitor. Tests for monitoring the effectiveness of treatment with a FXa variant include tests that directly or indirectly measure the ability to clot or that measure the activity of a direct FXa inhibitor. Non-limiting exemplary tests include prothrombin time or the related International Normalized Ratio, the prothrombinase-induced clotting time assay, thromboelastometry, thromboelastography, chromogenic anti-FXa assay, thrombin generation assay, level of prothrombin fragment 1 + 2, level of thrombin-antithrombin III complex, activated partial thromboplastin time, and partial thromboplastin time. Other tests are also possible within the knowledge of those of ordinary skill in the art.

According to some disclosures, reversing the effects of a direct FXa inhibitor in a subject by administering a FXa variant reduces bleeding in the subject. In some embodiments of the invention, FXa variant for use according to the invention reduces bleeding in a subject at least 10%, 20%, 30%, 40%, 50%, 60%, 70%, 80%, 90%, 95%, or 99% in the presence of a direct FXa inhibitor compared to absence of treatment with FXa variant. In other embodiments of the invention, FXa variant for use according to the invention reduces bleeding in a subject about 5%-10%, 10%-15%, 15%-20%, 20%-25%, 25%-30%, 30%-35%, 35%-40%, 40%-45%, 45%-50%, 50%-55%, 55%-60%, 60%-65%, 65%-70%, 70%-75%, 75%-80%, 80%-85%, 85%-90%, 90%-95%, or 95%-100%.

According to some disclosures, reversing the effects of a direct FXa inhibitor in a subject by administering a FXa variant reduces the activity of a direct FXa inhibitor in the subject. In some disclosures, treatment with FXa variant reduces activity of the direct FXa inhibitor in a subject at least 10%, 20%, 30%, 40%, 50%, 60%, 70%, 80%, 90%, 95%, or 99% in the presence of a direct FXa inhibitor compared to absence of treatment with FXa variant. In other disclosures, treatment with FXa variant reduces the activity of a direct FXa inhibitor in a subject about 5%-10%, 10%-15%, 15%-20%, 20%-25%, 25%-30%, 30%-35%, 35%-40%, 40%-45%, 45%-50%, 50%-55%, 55%-60%, 60%-65%, 65%-70%, 70%-75%, 75%-80%, 80%-85%, 85%-90%, 90%-95%, or 95%-100%.

Activity of a direct FXa inhibitor can be monitored using a chromogenic anti-FXa assay, such as that described in Asmis, et al., Thromb Res., 129:492-498 (2012), or Barrett, et al., Thromb Haemost. 104:1263-71 (2010), each of which are incorporated by reference herein.

According to some disclosures, reversing the effects of a direct FXa inhibitor in a subject by administering a FXa variant increases the amount of thrombin produced in the blood or plasma of the subject. In some disclosures, treatment with FXa variant increases thrombin production in a subject at least 10%, 20%, 30%, 40%, 50%, 60%, 70%, 80%, 90%, 95%, 100%, 1.5 fold, 2-fold, 3-fold, 4-fold, 5-fold, 6-fold, 7-fold, 10-fold, 15-fold, 20-fold, 25-fold, 30-fold, at least 50-fold, or more in the presence of a direct FXa inhibitor compared to the absence of an FXa variant. Thrombin production in the blood or plasma of a subject can be determined using the thrombin generation assay (TGA) or other technique familiar to those of ordinary skill in the art.

According to some disclosures, reversing the effects of a direct FXa inhibitor in a subject by administering a FXa variant increases clotting in the subject. In some disclosures, treatment with FXa variant increases clotting in a subject at least 10%, 20%, 30%, 40%, 50%, 60%, 70%, 80%, 90%, 95%, 100%, 1.5 fold, 2-fold, 3-fold, 4-fold, 5-fold, 6-fold, 7-fold, 10-fold, 15-fold, 20-fold, 25-fold, 30-fold, at least 50-fold, or more in the presence of a direct FXa inhibitor compared to the absence of an FXa variant.

According to some disclosures, reversing the effects of a direct FXa inhibitor in a subject by administering a FXa variant reduces clotting time in the subject. In some disclosures, treatment with FXa variant reduces clotting time in a subject at least 10%, 20%, 30%, 40%, 50%, 60%, 70%, 80%, 90%, 95%, or 99% in the presence of a direct FXa inhibitor compared to absence of treatment with FXa variant. In other disclosures, treatment with FXa variant reduces clotting time in a subject about 5%-10%, 10%-15%, 15%-20%, 20%-25%, 25%-30%, 30%-35%, 35%-40%, 40%-45%, 45%-50%, 50%-55%, 55%-60%, 60%-65%, 65%-70%, 70%-75%, 75%-80%, 80%-85%, 85%-90%, 90%-95%, or 95%-100%.

According to some disclosures, clotting time is determined by measuring the subject's prothrombin time (PT) which decreases as hemostasis is restored. PT is the amount of time it takes for serum to clot after addition of tissue factor. PT therefore measures the capability of the extrinsic clotting system to support clotting. PT can vary depending on the particular reagents a lab uses to run the test, but a normal PT is about 11 to 13 seconds. Clotting time can also be expressed using the International Normalized Ratio (INR), which eliminates lab to lab variability in clotting time measurements. Using the INR, a ratio of 0.8 to 1.1 indicates normal clotting. PT or INR can be determined at a predetermined time after a FXa variant is administered to a subject in need of reversal of the effects of a direct FXa inhibitor.

In some disclosures, treatment with a FXa variant to reverse the effects of a direct FXa inhibitor reduces the PT of a subject to about 25 seconds, 24 seconds, 23 seconds, 22 seconds, 21 seconds, 20 seconds, 19 seconds, 18 seconds, 17 seconds, 16 seconds, 15 seconds, 14 seconds, 13 seconds, 12 seconds, 11 seconds, 10 seconds, or less. In other disclosures, treatment with a FXa variant reduces the INR or a subject to about 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.1, 1.0, 0.9, 0.8, 0.7, or less. According to other disclosures, treatment with FXa variant reduces PT or INR in a subject about 5%-10%, 10%-15%, 15%-20%, 20%-25%, 25%-30%, 30%-35%, 35%-40%, 40%-45%, 45%-50%, 50%-55%, 55%-60%, 60%-65%, 65%-70%, 70%-75%, 75%-80%, 80%-85%, 85%-90%, 90%-95%, or 95%-100%.

Prothrombin time can be measured at a predetermined after administration of a FXa variant. Thus, in some non-limiting disclosures, PT is measured 15 mins, 20 mins, 30 mins, 40 mins, 45 mins, 50 mins, 60 mins or more after administration of FXa. Other times are also possible according to the knowledge of those of ordinary skill in the art.

Clotting time can also be measured using the one-step prothrombinase-induced clotting time (PiCT) assay as described in Graff, et al., Monitoring effects of direct FXa-inhibitors with a new one-step prothrombinase-induced clotting time (PiCT) assay: comparative in vitro investigation with heparin, enoxaparin, fondaparinux and DX 9065a, Int J Clin Pharmacol Ther., 45:237-43 (2007) and Harder, et al., Monitoring direct FXa-inhibitors and fondaparinux by Prothrombinase-induced Clotting Time (PiCT): relation to FXa-activity and influence of assay modifications, Thromb Res., 123:396-403 (2008), each of which are incorporated by reference.

In yet other disclosures, the methods of thromboelastometry or thromboelastography may be used to analyze clot formation or clotting time.

According to some disclosures, reversing the effects of a direct FXa inhibitor in a subject by administering a FXa variant increases the level of prothrombin fragment 1 + 2 (PF1 + 2) in the blood or plasma of the subject. In some disclosures, treatment with FXa variant increases PF1 + 2 in a subject at least 10%, 20%, 30%, 40%, 50%, 60%, 70%, 80%, 90%, 95%, 100%, 1.5-fold, 2-fold, 3-fold, 4-fold, 5-fold, 6-fold, 7-fold, 10-fold, 15-fold, 20-fold, 25-fold, 30-fold, at least 50-fold, or more in the presence of a direct FXa inhibitor compared to the absence of an FXa variant.

According to some disclosures, reversing the effects of a direct FXa inhibitor in a subject by administering a FXa variant increases the level of thrombin-antithrombin III complex (TAT) in the blood or plasma of the subject. In some disclosures, treatment with FXa variant increases TAT in a subject at least 10%, 20%, 30%, 40%, 50%, 60%, 70%, 80%, 90%, 95%, 100%, 1.5-fold, 2-fold, 3-fold, 4-fold, 5-fold, 6-fold, 7-fold, 10-fold, 15-fold, 20-fold, 25-fold, 30-fold, at least 50-fold, or more in the presence of a direct FXa inhibitor compared to the absence of an FXa variant.

According to some disclosures, reversing the effects of a direct FXa inhibitor in a subject by administering a FXa variant reduces activated partial thromboplastin time (aPTT) in the subject. In some disclosures, treatment with FXa variant reduces activated partial thromboplastin time (aPTT) in a subject at least 10%, 20%, 30%, 40%, 50%, 60%, 70%, 80%, 90%, 95%, or 99% in the presence of a direct FXa inhibitor compared to absence of treatment with FXa variant. In other disclosures, treatment with FXa variant reduces aPTT in a subject about 5%-10%, 10%-15%, 15%-20%, 20%-25%, 25%-30%, 30%-35%, 35%-40%, 40%-45%, 45%-50%, 50%-55%, 55%-60%, 60%-65%, 65%-70%, 70%-75%, 75%-80%, 80%-85%, 85%-90%, 90%-95%, or 95%-100%.

According to some disclosures, reversing the effects of a direct FXa inhibitor in a subject by administering a FXa variant reduces partial thromboplastin time (PTT) in the subject. In some disclosures, treatment with FXa variant reduces partial thromboplastin time (PTT) in a subject at least 10%, 20%, 30%, 40%, 50%, 60%, 70%, 80%, 90%, 95%, or 99% in the presence of a direct FXa inhibitor compared to absence of treatment with FXa variant. In other disclosures, treatment with FXa variant reduces PTT in a subject about 5%-10%, 10%-15%, 15%-20%, 20%-25%, 25%-30%, 30%-35%, 35%-40%, 40%-45%, 45%-50%, 50%-55%, 55%-60%, 60%-65%, 65%-70%, 70%-75%, 75%-80%, 80%-85%, 85%-90%, 90%-95%, or 95%-100%.

In other disclosures, clinical endpoints can be relied upon to determine if hemostasis has been adequately restored in a subject treated with a FXa variant to reverse the effects of a direct FXa inhibitor. For example, where a subject presents with acute bleeding, clinical hemostatic efficacy can be scored "very good" where prompt cessation of existing bleeding occurs after treatment with FXa variant; "satisfactory" where there is a 1-2 hr delay in bleeding cessation; "questionable" where there is a >2 hr delay in bleeding cessation; and "none" where an effect on bleeding is absent. Where treatment with FXa variant is determined to be less than satisfactory, then an additional dose of FXa variant can be administered to effect adequate hemostasis. In a further example, where a subject is undergoing an interventional procedure, clinical hemostatic efficacy can be scored "very good" where normal hemostasis is attained during the procedure; "satisfactory" where intraprocedural hemostasis is mildly abnormal as judged by quantity or quality of blood loss (e.g., slight oozing); "questionable" where intraprocedural hemostasis is moderately abnormal as judged by quantity or quality of blood loss (e.g., controllable bleeding); and "none" where intraprocedural hemostasis is severely abnormal as judged by quantity or quality of blood loss (e.g., severe refractory hemorrhage).

A therapeutically effective dose of a direct FXa inhibitor depends upon numerous factors that are well known to a medical practitioner of skill in the art. A typical therapeutic plasma concentration of rivaroxaban is about 500 nM. However, according to the invention, an FXa variant for use according to the invention can be administered to counteract lower or higher concentrations of inhibitor. The plasma concentration of rivaroxaban in a subject to be treated with an FXa variant may be lower or higher than the typical therapeutic concentration, for example according to the invention about 100 nM, about 200 nM, about 300 nM, about 400 nM, about 500 nM, about 600 nM, about 700 nM, according to the disclosure about 800 nM, about 900 nM or about 1,000 nM.

A typical therapeutic plasma concentration of apixaban is about 250 nM. In certain embodiments, the FXa variant for use according to the invention is administered to a subject with a plasma concentration of apixaban of about 100 nM, about 200 nM, about 300 nM, about 400 nM, according to the disclosure about 500 nM, about 600 nM, about 700 nM, about 800 nM, about 900 nM or about 1,000 nM.

Likewise, according to the disclosure, an FXa variant can be used to counteract a direct FXa inhibitor in cases of overdose, such as when the plasma concentration of the inhibitor is at least 20%, at least 30%, at least 40%, at least 50%, at least 60%, at least 70%, at least 80%, at least 90%, at least 95%, at least 99%, or at least 1.5 fold, at least 2-fold, at least 3-fold, at least 4-fold, at least 5-fold, at least 6-fold, at least 7-fold, at least 10-fold, at least 15-fold, at least 20-fold, at least 25-fold, at least 30-fold, or at least 50-fold higher than the typical therapeutic plasma concentration.

The FXa variants are surprisingly effective in counteracting a direct FXa inhibitor at a plasma concentration that is lower than the plasma concentration of the direct FXa inhibitor. According to the disclosure, the FXa variant counters the effect of a direct FXa inhibitor at a plasma concentration ratio of variant to inhibitor of about 1 to 10, about 1 to 25, about 1 to 50, about 1 to 100, about 1 to 250, about 1 to 500, about 1 to 1,000, about 1 to 2,500, about 1 to 5,000 or about 1 to 10,000. In certain embodiments, the FXa variant for use according to the invention counters the effect of a direct FXa inhibitor at a plasma concentration of at least 10-fold, at least 25-fold, at least 50-fold, at least 100-fold, at least 250-fold, at least 500-fold, at least 1,000-fold, at least 2,500-fold, at least 5,000-fold, or at least 10,000-fold lower than the plasma concentration of the direct FXa inhibitor.

In other embodiments of the invention, the plasma concentration of an FXa variant sufficient to reverse the effect of a direct FXa inhibitor is calculated by multiplying the plasma concentration of the direct inhibitor by a conversion factor ranging from about 0.1 x 10⁻⁴ to about 1000 x 10⁻⁴, according to the disclosure about 4 x 10⁻⁴ to about 40 x 10⁻⁴, about 20 x 10⁻⁴ to about 200 x 10⁻⁴, or other ranges. In yet other disclosures, the conversion factor can be about 0.1 x 10⁻⁴, 0.5 x 10⁻⁴, 1 x 10⁻⁴, 2 x 10⁻⁴, 3 x 10⁻⁴, 4 x 10⁻⁴, 5 x 10⁻⁴, 6 x 10⁻⁴, 7 x 10⁻⁴, 8 x 10⁻⁴, 9 x 10⁻⁴, 10 x 10⁻⁴, 11 x 10⁻⁴, 12 x 10⁻⁴, 13 x 10⁻⁴, 14 x 10⁻⁴, 15 x 10⁻⁴, 16 x 10⁻⁴, 17 x 10⁻⁴, 18 x 10⁻⁴, 19 x 10⁻⁴, 20 x 10⁻⁴, 21 x 10⁻⁴, 22 x 10⁻⁴, 23 x 10⁻⁴, 24 x 10⁻⁴, 25 x 10⁻⁴, 26 x 10⁻⁴, 27 x 10⁻⁴, 28 x 10⁻⁴, 29 x 10⁻⁴, 30 x 10⁻⁴, 31 x 10⁻⁴, 32 x 10⁻⁴, 33 x 10⁻⁴, 34 x 10⁻⁴, 35 x 10⁻⁴, 36 x 10⁻⁴, 37 x 10⁻⁴, 38 x 10⁻⁴, 39 x 10⁻⁴, 40 x 10⁻⁴, 45 x 10⁻⁴, 50 x 10⁻⁴, 55 x 10⁻⁴, 60 x 10⁻⁴, 65 x 10⁻⁴, 70 x 10⁻⁴, 75 x 10⁻⁴, 80 x 10⁻⁴, 85 x 10⁻⁴, 90 x 10⁻⁴, 95 x 10⁻⁴, 100 x 10⁻⁴, 110 x 10⁻⁴, 120 x 10⁻⁴, 130 x 10⁻⁴, 140 x 10⁻⁴, 150 x 10⁻⁴, 160 x 10⁻⁴, 170 x 10⁻⁴, 180 x 10⁻⁴, 190 x 10⁻⁴, 200 x 10⁻⁴, 250 x 10⁻⁴, 300 x 10⁻⁴, 350 x 10⁻⁴, 400 x 10⁻⁴, 450 x 10⁻⁴, 500 x 10⁻⁴, 550 x 10⁻⁴, 600 x 10⁻⁴, 650 x 10⁻⁴, 700 x 10⁻⁴, 750 x 10⁻⁴, 800 x 10⁻⁴, 850 x 10⁻⁴, 900 x 10⁻⁴, 950 x 10⁻⁴, or 1000 x 10⁻⁴, and ranges among these numbers. Plasma concentration of FXa direct inhibitor can be measured according to the knowledge of the skilled artisan, for example, by radio-immuno assay (RIA) or other method.

Achieving a target plasma concentration of FXa variant sufficient to reverse overdose of a direct FXa inhibitor is within the knowledge of those ordinarily skilled in the art. In a non-limiting example, estimates of relevant pharmacokinetic parameters, such as subject plasma volume or other parameters, can be made based on upon subject sex, height and weight, or other factors, and used to calculate how much FXa variant needs be administered to achieve the target concentration. After administering FXa variant, plasma concentrations can be monitored according to the knowledge of those ordinarily skilled in the art and this information used to maintain the concentration in any desired range.

The compositions and methods of the disclosure include a "therapeutically effective amount" or a "prophylactically effective amount" of an FXa variant. A "therapeutically effective amount" refers to an amount effective, at dosages and for periods of time necessary, to achieve the desired therapeutic result. A therapeutically effective amount of the FXa variant may vary according to factors such as the disease state, age, sex, and weight of the individual, and the ability of the FXa variant to elicit a desired response in the individual. A therapeutically effective amount is also one in which any toxic or detrimental effects of the FXa variant are outweighed by the therapeutically beneficial effects. A "prophylactically effective amount" refers to an amount effective, at dosages and for periods of time necessary, to achieve the desired prophylactic result. For example, a dose may be given prior to a planned surgery.

Dosage regimens can be adjusted to provide the optimum desired response (e.g., a therapeutic or prophylactic response). For example, a single bolus can be administered, several divided doses can be administered over time or the dose can be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the disclosure are dictated by and directly dependent on (a) the unique characteristics of the FXa variant and the particular therapeutic or prophylactic effect to be achieved, and (b) the limitations inherent in the art of compounding such an FXa variant for the treatment of individuals.

In certain embodiments of the invention, a therapeutically or prophylactically-effective amount of an FXa variant administered is about 0.0001 to 50 mg/kg, about 0.001 to 50 mg/kg, about 0.001 to 5 mg/kg, about 0.001 to 0.5 mg/kg, about 0.001 to 0.05 mg/kg, about 0.01 to 5 mg/kg or about 0.01 to 0.5 mg/kg.

In certain embodiments of the invention, a therapeutically or prophylactically-effective serum concentration of an FXa variant of the disclosure is about 0.0003 to 300 nM, about 0.003 to 300 nM, about 0.03 to 300 nM, about 0.003 to 30 nM, about 0.03 to 30 nM or about 0.3 to 3 nM. The concentration of the FXa variant, for example in blood or plasma, may be measured by any method known in the art.

It is to be noted that dosage values may vary with FXa inhibitor concentration. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

Another aspect of the present disclosure provides kits comprising an FXa variant or a composition comprising such an FXa variant. A kit may include, in addition to the FXa variant or composition, diagnostic or additional therapeutic agents. A kit can also include instructions for use in a therapeutic method, as well as packaging material such as, but not limited to, ice, dry ice, styrofoam, foam, plastic, cellophane, shrink wrap, bubble wrap, cardboard and starch peanuts. In one embodiment, the kit includes the FXa variant or a composition comprising it and one or more therapeutic agents that can be used in a method described herein.

According to the invention the FXa variant may be administered, for example in a composition comprising it, once or multiple times to a subject until adequate hemostasis is restored or the direct FXa inhibitor or inhibitors are no longer effective. According to the disclosure where multiple administrations are used they may administered hourly, daily, or at any other appropriate interval, including for example multiple daily doses. Multiple doses may be administered on a schedule such as every 10 minutes, every 15 minutes, every 20 minutes, every 30 minutes, every hour, every two hours, every three hours, every four hours, three times daily, twice daily, once daily, once every two days, once every three days, and once weekly. The FXa variant may also be administered continuously, e.g. via a minipump. The FXa variant may be administered, for example, via a parenteral route (e.g., intravenously, subcutaneously, intraperitoneally, or intramuscularly). The FXa variant will generally be administered as part of a pharmaceutical composition as described below.

In another embodiment of the invention, the FXa variant may be co-administered with another procoagulant including another FXa variant, Factor IX, Factor Xla, Factor XIIa, Factor VIII, Factor VIIa, FEIBA and prothrombin complex concentrate (PCC).

Co-administration of an FXa variant of the disclosure with an additional therapeutic agent (combination therapy) encompasses administering a pharmaceutical composition comprising the FXa variant and the additional therapeutic agent, as well as administering two or more separate pharmaceutical compositions, i.e., one comprising the FXa variant and the other(s) comprising the additional therapeutic agent(s). Co-administration or combination therapy further includes administering the FXa variant and additional therapeutic agent(s) simultaneously or sequentially, or both. For instance, the FXa variant may be administered once every three days, while the additional therapeutic agent is administered once daily at the same as the FXa variant, or at a different time. An FXa variant may be administered prior to or subsequent to treatment with the additional therapeutic agent. Similarly, administration of an FXa variant of the disclosure may be part of a treatment regimen that includes other treatment modalities including surgery. The combination therapy may be administered to prevent recurrence of the condition. The combination therapy may be administered from multiple times hourly to weekly. The administrations may be on a schedule such as every 10 minutes, every 15 minutes, every 20 minutes, every 30 minutes, every hour, every two hours, every three hours, every four hours, three times daily, twice daily, once daily, once every two days, once every three days, once weekly, or may be administered continuously, *e.g*. via a minipump. The combination therapy may be administered, for example, via a parenteral route (*e.g.*, intravenously, subcutaneously, intraperitoneally, or intramuscularly).

In a further aspect, the invention provides a composition comprising an FXa variant for use according to the invention in counteracting a direct FXa inhibitor in a subject. The composition may comprise a pharmaceutically acceptable carrier, vehicle or other ingredients that are physiologically compatible. Non-limiting examples of such carriers, vehicles and other ingredients include solvents (e.g., water, ethanol, saline, phosphate buffered saline), detergents, surfactants, dispersion media, coatings, antibacterial or antifungal agents, isotonifying agents, absorption delaying agents, sugars (e.g., sucrose, dextrose, lactose), polyalcohols (e.g., glycerol, mannitol, sorbitol), salts (e.g., sodium chloride, potassium chloride), wetting agents, emulsifying agents, preservatives, buffers, and agents capable of enhancing the stability or effectiveness of the FXa variant.

A composition for use according to the disclosure may be in any suitable form for administration to a subject, such as liquid solutions (*e.g*., injectable and infusible solutions). Compositions can be provided in a pre-mixed format ready for administration to a subject, for example, in a vial or pre-filled syringe. Such formats do not require reconstitution with diluent before administration. Alternatively, compositions can be provided in lyophilized form requiring reconstitution with diluent (e.g., sterile water or saline) before administration. If the latter, diluent can be provided with the lyophilisate in a separate container. According to the knowledge of those of ordinary skill in the art, compositions can be formulated for storage under refrigeration or at room temperature. The form of the composition depends, at least in part, on the intended mode of administration. In certain disclosures, the mode of administration is parenteral, including for example intravenous, subcutaneous, intraperitoneal, or intramuscular administration.

Therapeutic compositions typically must be sterile and stable under the conditions of manufacture and storage. The composition can be formulated as a solution, microemulsion, dispersion, in liposomes, or other ordered structure suitable to high drug concentration. Sterile injectable solutions can be prepared by incorporating the FXa variant in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

It is further contemplated by the present disclosure that any of the compositions herein may be administered to a subject being treated with a direct FXa inhibitor.

It is understood that the examples and embodiments described herein are for illustrative purposes only and that various modifications or changes in light thereof will be apparent to persons skilled in the art and are to be included within the and can be made without departing from the true scope of the disclosure.

### EXAMPLES

### Example 1 - FXa^{I16L} Sensitivity Toward Rivaroxaban

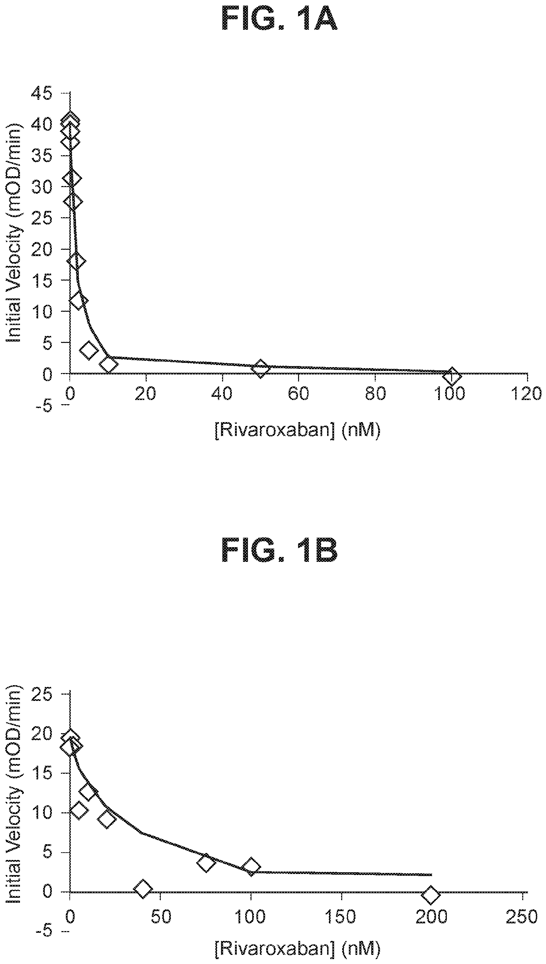

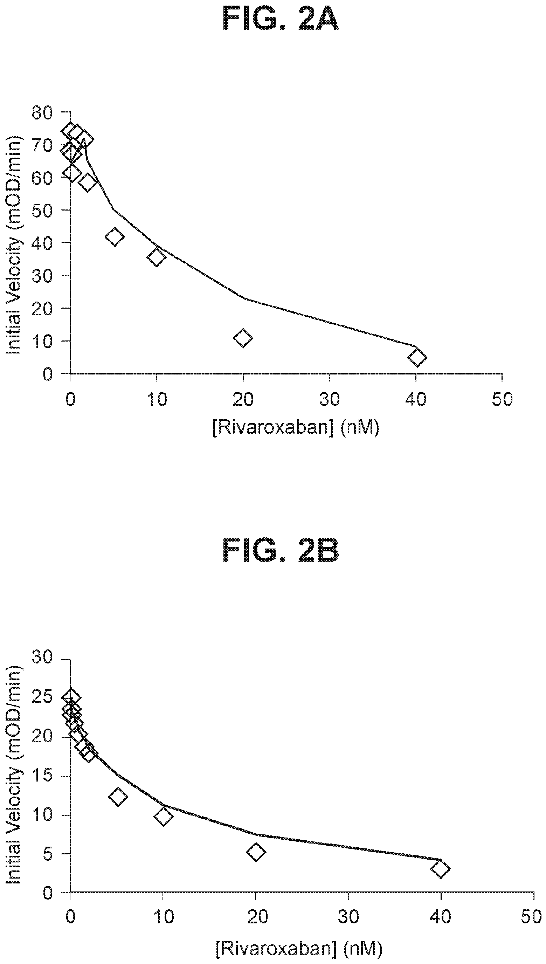

To test the sensitivity of FXa^{I16L} toward rivaroxaban, inhibition assays were established. Rivaroxaban was an efficient inhibitor of wild-type FXa exhibiting an inhibition constant (Kᵢ) of 0.582 nM **(****Figure 1A****).** Due to the zymogen-like nature of FXa^{I16L}, rivaroxaban bound with a -15-fold reduced affinity to this variant (Kᵢ = 9.3 nM) **(****Figure 1B****).** In contrast, when the variant was assembled in the prothrombinase complex (i.e. upon addition of FVa and phospholipid vesicles), the Kᵢ for rivaroxaban was nearly restored to a value comparable to the wild-type enzyme (wt FXa, Kᵢ = 2.67 nM **(****Figure 2A****);** FXa^{I16L}, Kᵢ = 3.4 nM **(****Figure 2B****).**

### Example 2 - FXa Variants Counteract Rivaroxaban and Apixaban

The thrombin generation assay (TGA) was used to assess whether the zymogen-like FXa variants can reverse the effects of direct FXa inhibitors in a more physiologic environment. The TGA measures thrombin production in plasma over time following the initiation of coagulation and was performed as previously described (See Bunce et al., (2011) Blood 117, 290-298, incorporated by reference herein in their entirety). Thrombin generation in normal human plasma was measured for 90 min at 37°C in the presence or absence of 500 nM rivaroxaban. To evaluate if FXa^{I16L} can reverse the effect of rivaroxaban, increasing amounts of FXa^{I16L} was added to plasma containing 500 nM rivaroxaban. Thrombin generation was initiated with 2.0 pM tissue factor/4 uM phospholipid as well as CaCl₂ and a thrombin fluorogenic substrate.

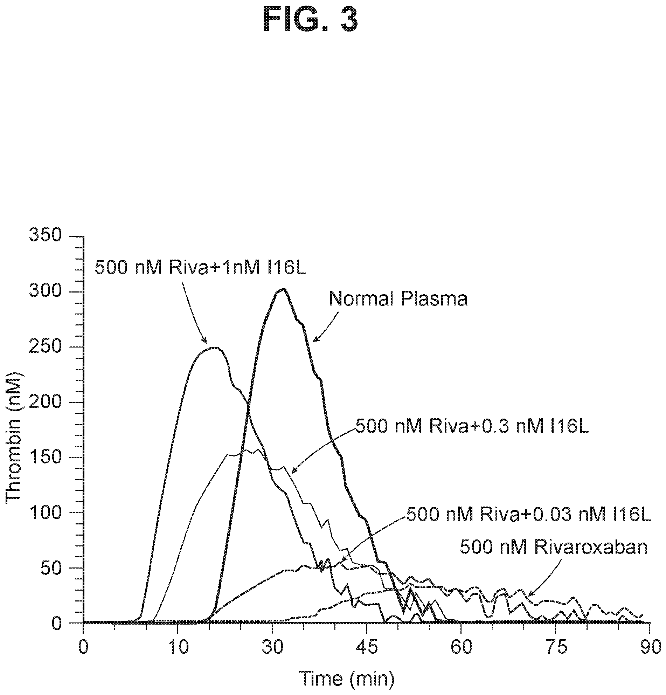

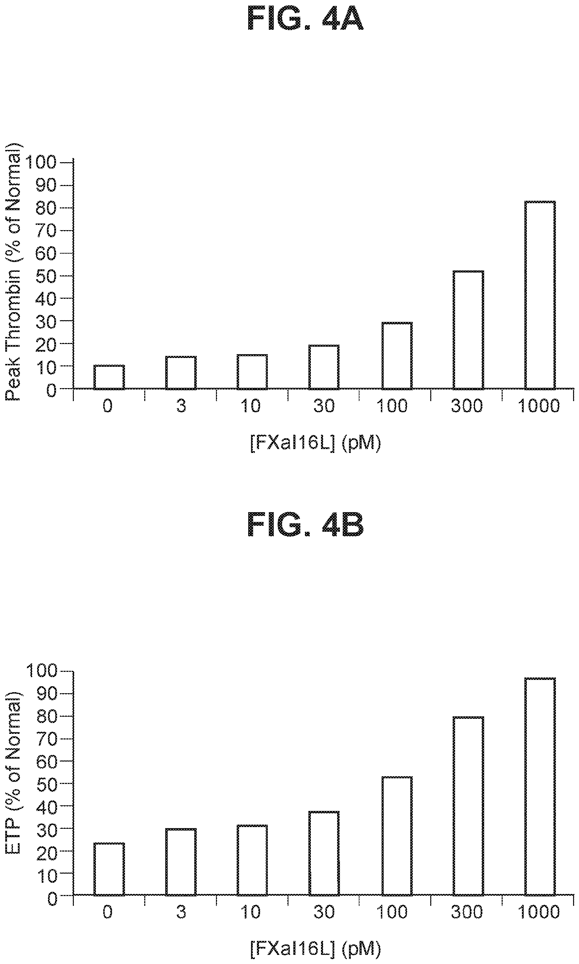

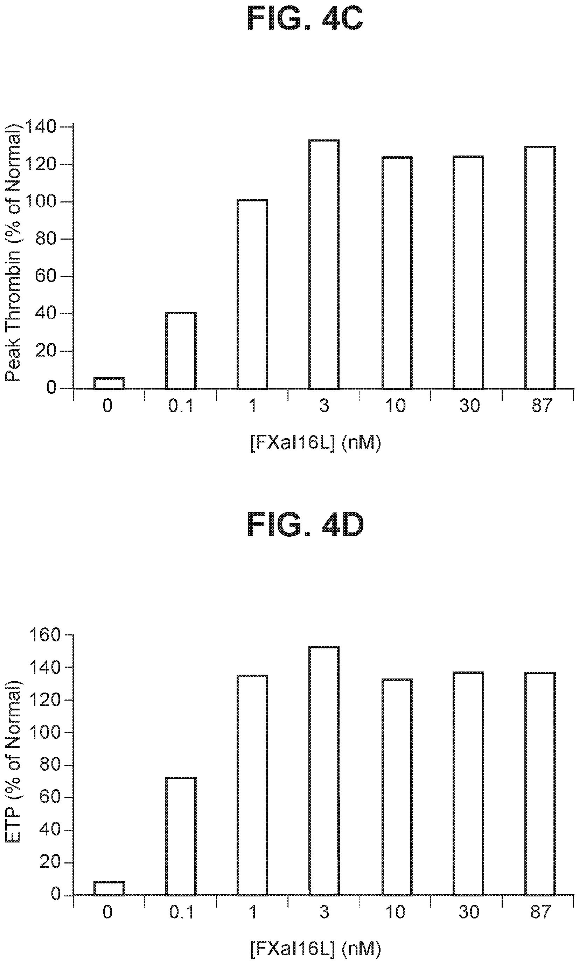

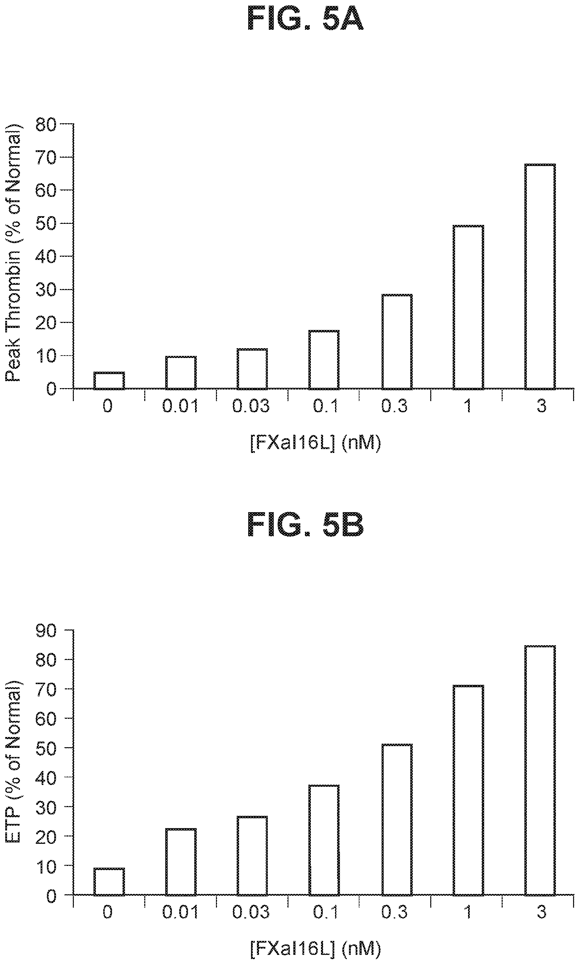

The data demonstrated that the thrombin generation profile of plasma in the presence of 500 nM rivaroxaban was substantially reduced compared to plasma in the absence of rivaroxaban. In contrast, increasing concentrations of FXa^{I16L} from 0.03 to 1 nM restored thrombin generation **(****Figure 3****).** These data show that unexpectedly low concentrations of FXa^{I16L} in the nanomolar and subnanomolar range can reverse the effects of the inhibitor. Dose response analysis of FXa^{I16L} in the presence of 500 nM rivaroxaban (a typical therapeutic plasma concentration) shows that the peak height of thrombin generation **(****Figure 4A** **and** **C****)** and total thrombin produced (ETP) **(****Figure 4** **B and D)** essentially reached a maximum and was completely restored to normal levels between 1-3 nM of FXa^{I16L} under these conditions. Further experiments showed that even in the presence of high concentration of rivaroxaban (7.5 µM; supratherapeutic), FXa^{I16L} was still quite effective at a relatively low dose (≤ 3.0 nM) in restoring peak thrombin **(****Figure 5A****)** as well as total thrombin generated **(****Figure 5B****).**

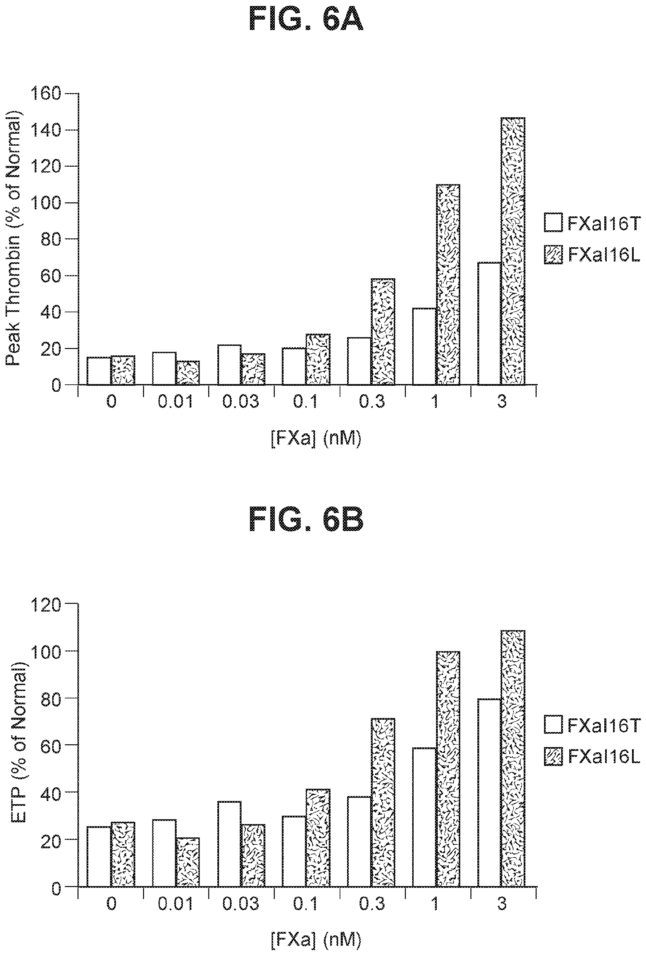

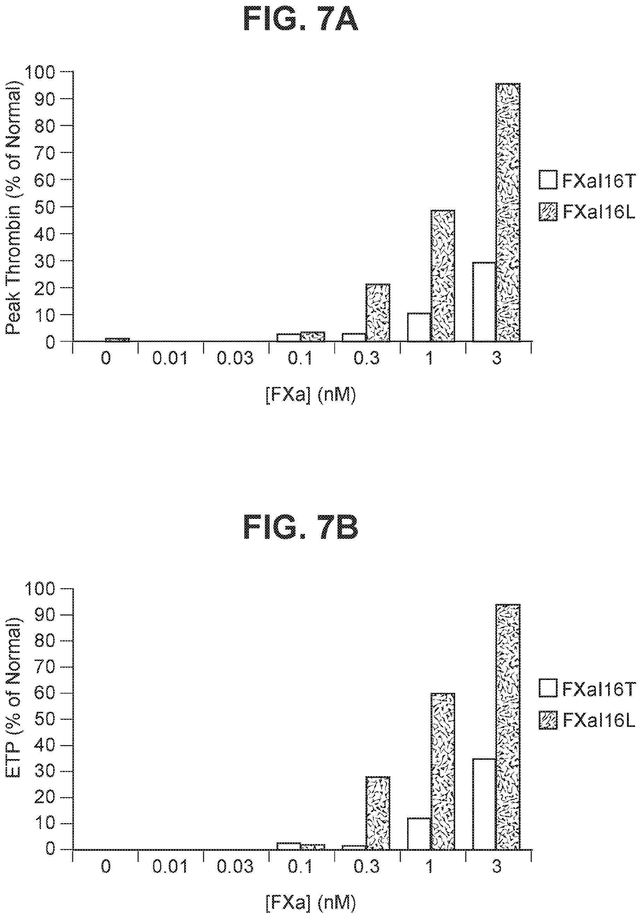

Similar experiments were also performed to evaluate whether FXa zymogen-like variants could reverse the effects of another direct FXa inhibitor, apixaban. In these experiments, the effectiveness of FXa^{I16L} with another zymogen-like FXa variant, FXa^{I16T}, was also compared. FXa^{I16T} is similar to FXa^{I16L}, however it has intrinsically less activity, has a longer plasma half-life, and has -3-5-fold reduced activity compared to FXa^{I16L} when assembled in the prothrombinase complex. Consistent with the rivaroxaban data, FXa^{I16L} could restore peak thrombin **(****Figure 6A****)** and total thrombin **(****Figure 6B****)** generated in the presence of 250 nM apixaban (a typical therapeutic plasma concentration) in a dose-dependent manner, which appears to reach a maximum between 1-3 nM of FXa^{I16L}. FXa^{I16T} was also effective at reversing the effects of apixaban; however, it appears that higher concentrations of this variant are needed to fully restore thrombin generation **(****Figure 6****)**. Both variants were still effective even in the presence of a higher concentration of apixaban (2 µM). However, under these conditions it appears that higher concentrations of both variants are needed to fully restore thrombin generation **(****Figure 7A and B****).**

### Example 3 - FXa^{I16L} Counteracts Rivaroxaban and Apixaban in Whole Blood

Whole blood thromboelastometry was used to assess the ability of the FXa^{I16L} variant to reverse the effects of the direct FXa inhibitors in whole blood. In this system, blood is drawn from healthy volunteers. The first 2 mL of blood were discarded and the subsequent 5 mL of blood was collected into a vacutainer (BD, Franklin Lakes, NJ). Corn trypsin inhibitor and sodium citrate were in the collection tube, prior to collection of the blood sample, to achieve a final concentration of 0.105 M citrate and 25 µg/mL corn trypsin inhibitor (Haematologic Technologies, Burlington, VT) in the blood. Two sets of reactions were analyzed for each donor. The initial reaction initiated 5 minutes post blood collection. The second reaction initiated 1 hour post initiation of first reaction (1 hour 5 minutes after collection).

Blood was analyzed using Thromboelastometry ROTEM®delta (Tem International GmbH, Munich, Germany). For the reaction: (1) 6 µL of vehicle, protein, and/or inhibitor were added to the empty cup, (2) 20 µl of 0.2 M CaCl₂ (final concentration 11.6 mM), and (3) 20 µl of Innovin (final concentration in reaction 1:10,000; source of tissue factor) were added to the cup. Whole blood collected as described above was added to the reaction (300 µL) and recordings were allowed to proceed for approximately 30-60 minutes. The data collected was analyzed using the manufacture's software (Rotem Gamma Software Version 1.1.1).

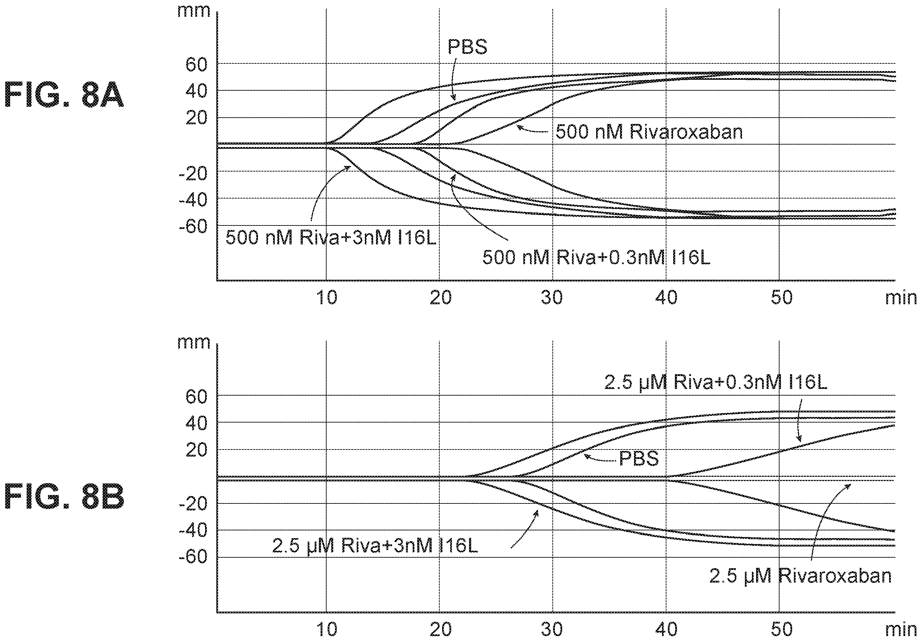

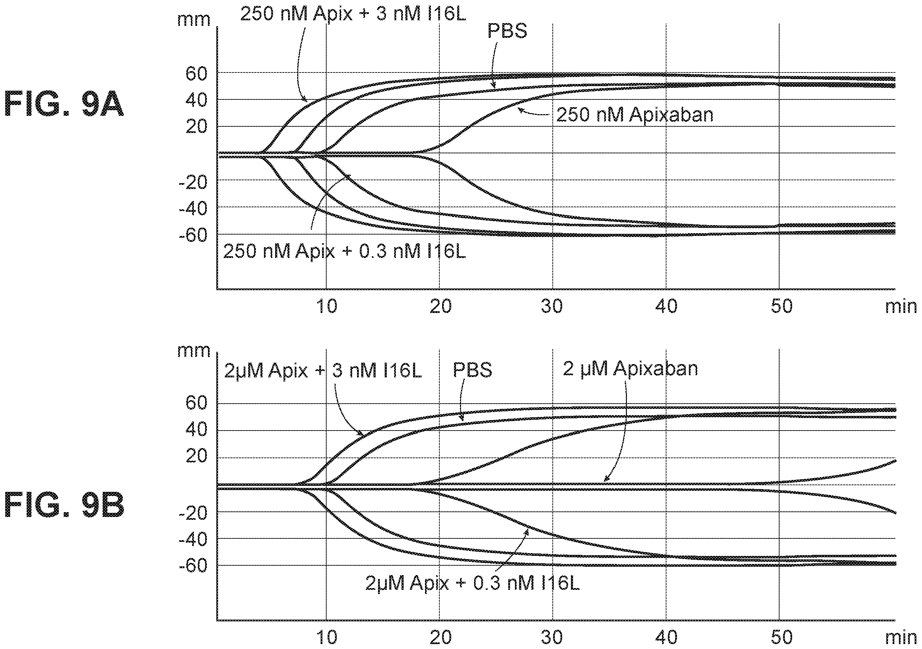

The ability of FXa^{I16L} to accelerate whole blood clot formation in the presence of rivaroxaban or apixaban was examined using rotational thromboelastometry (ROTEM). Both direct FXa inhibitors alone and at two different concentrations have a substantial effect on whole blood clot formation: at low doses (therapeutic concentrations), whole blood clot formation is partially eliminated **(****Figures 8A** **and** **Figure 9A****),** while at high doses (supratherapeutic concentrations), whole blood clot formation is almost completely eliminated **(****Figures 8B** **and** **Figure 9B****).** The effects of either rivaroxaban or apixaban on whole blood clot formation could be reversed by FXa^{I16L}. In the presence of either 500 nM rivaroxaban or 250 nM apixaban, 0.3 nM FXa^{I16L} could fully or nearly fully restore whole blood coagulation **(****Figures 8A** **and** **Figure 9A****).** When higher concentrations of the direct FXa inhibitors were used (∼ 2 uM), 0.3 nM FXa^{I16L} partially restored whole blood coagulation and 3 nM FXa^{I16L} fully restored it **(****Figures 8B** **and** **Figure 9B****).** These data demonstrated that an FXa zymogen-like variant can effectively reverse the anticoagulant effect of rivaroxaban or apixaban in plasma-based and whole-blood coagulation assays at both therapeutic and supratherapeutic concentrations of the inhibitor.

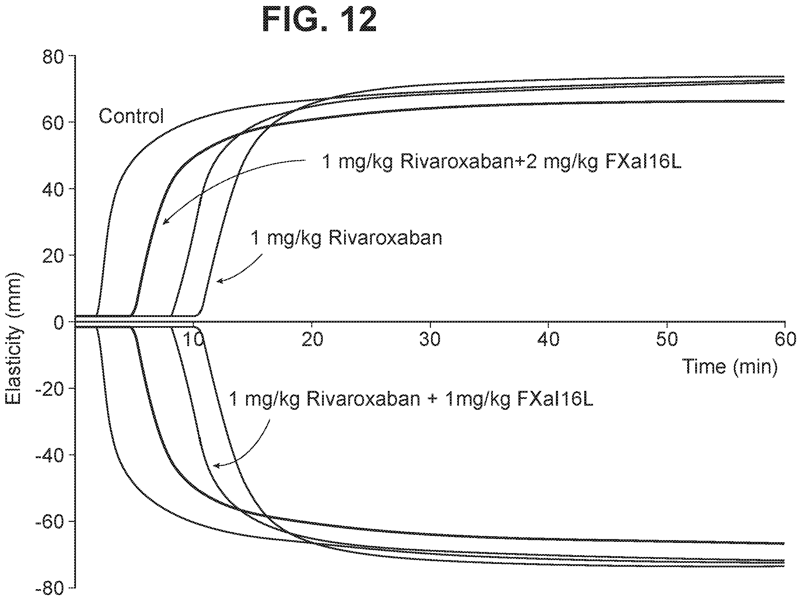

The results of these studies were confirmed and extended by testing if FXa^{I16L} could counteract the anti-coagulant effect of rivaroxaban when both agents were administered in vivo. In these experiments, C57BL/6 mice were infused with rivaroxaban (1 mg/kg) or buffer via the tail vein. Mice were then prepared to expose the jugular vein and the vena cava. Approximately 10 min later FXa^{I16L} (1 or 2 mg/kg) was infused by direct injection into the jugular vein. Five minutes post injection blood was collected via the vena cava into citrate and corn trypsin inhibitor. Collected blood was then analyzed by ROTEM using dilute tissue factor (Innovin, 1:42,000 dilution). Whole blood from mice administered buffer only clotted by about 2 min **(****Figure 12****).** Administration of 1 mg/kg rivaroxaban substantially prolonged the clot time to about 10 min **(****Figure 12****).** Further administration of FXa^{I16L} shortened clotting time in the presence of rivaroxaban in a dose dependent manner **(****Figure 12****)**.

### Example 4 - FXa^{I16L} Counteracts Rivaroxaban in a Thrombin Generation Assay

The effect of FXa^{I16L} on reversing rivaroxaban in plasma was examined in a thrombin generation assay (TGA) using the calibrated automated thrombography (CAT) system (Thrombinoscope BV, Maastricht, The Netherlands). Normal human plasma was obtained from George King Biomedical (Overland Park, KS). In the reaction, 20 µL of PPP-Reagent LOW containing 4 µM phospholipids and 1 pM tissue factor was added to 70 µL of pooled citrated normal human plasma (treated with 250 nM rivaroxaban, within the therapeutic plasma concentration range) in an Immulon 2HB round bottom 96 well plate with reactions duplicated. Immediately preceding reaction initiation, 10 µL of vehicle or FXa^{I16L} was added to plasma at final concentrations ranging from 0.03125 nM to 0.5 nM FXa^{I16L}, given a 120 µL total reaction volume. Reactions were initiated by addition of 20 µL FluCa buffer containing calcium chloride and fluorogenic substrate. Fluorescence of plasma reactions was read at 37°C at 20 second intervals on a Fluoroskan Ascent fluorometer and compared to those of reference thrombin calibrator reactions to determine thrombin concentrations. The intensity of the fluorescence signal (FU) was continuously monitored at 37°C using the CAT. Thrombin generation curves (nM thrombin vs. time) were analyzed to extract lag time, peak height, time to peak, and the area under the curve representing the endogenous thrombin potential (ETP) using the Thromboscope software (Thrombinoscope BV version).

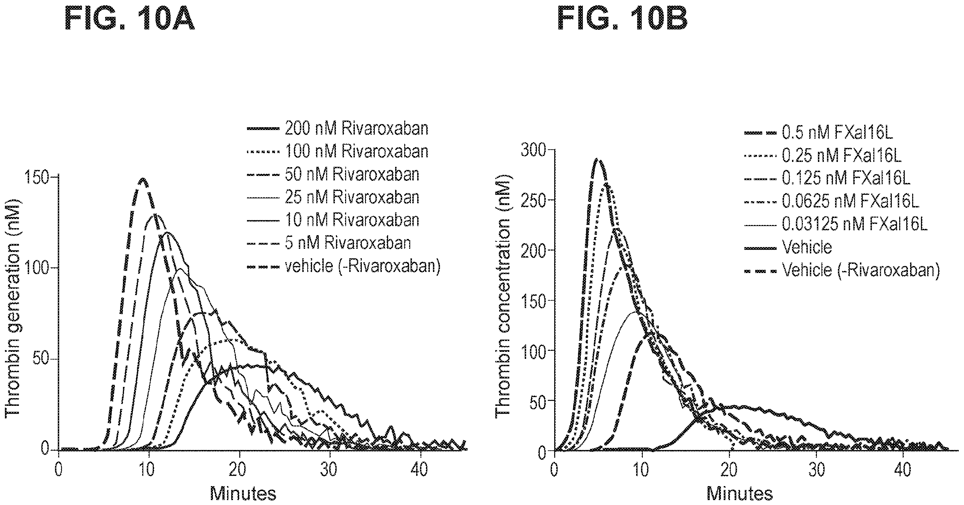

A dose dependent inhibition of thrombin generation in normal human plasma was observed with *in vitro* rivaroxaban treatment (5-200 nM) **(****Figure 10A****).** Rivaroxaban resulted in an increase in the lag time coupled with a decrease in the peak thrombin and a decrease in the ETP. The addition of FXa^{I16L} to rivaroxaban (250 nM) inhibited human plasma resulted in a dose dependent reversal of thrombin inhibition **(****Figure 10B****):** peak thrombin generation was restored, the lag phase was shorter, and the ETP increased. At a low dose of 0.03125 nM FXa^{I16L}, thrombin generation was restored to levels comparable to vehicle treated normal human plasma.

### Example 5 - FXa^{I16L} Counteracts Rivaroxaban in a Mouse Tail Clip Bleeding Model

The ability of FXa^{I16L} to overcome the effects of rivaroxaban *in vivo* was assessed in an acute bleeding model in normal mice. The results demonstrated that a zymogen-like FXa variant could reverse the anticoagulant effect of a direct FXa inhibitor.

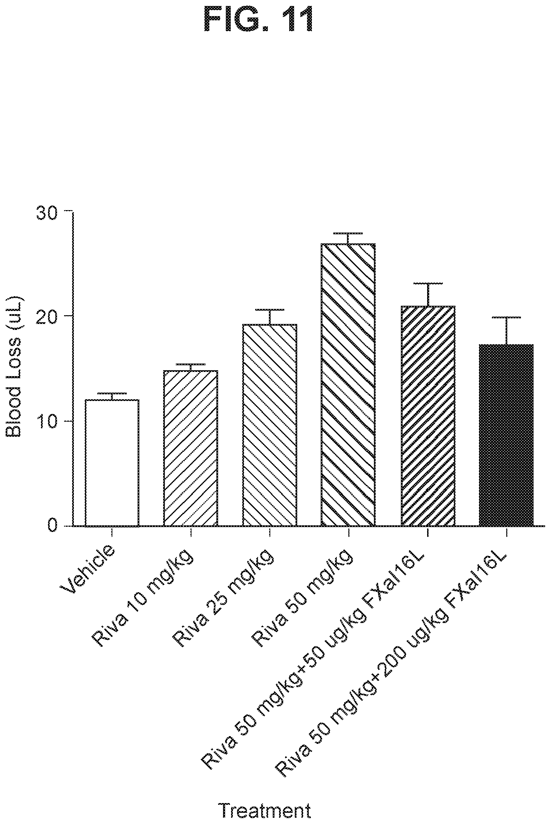

To establish a dose of rivaroxaban that would prolong bleeding, male C57BI/6 mice (The Jackson Laboratory, Bar Harbor, ME) received a single intravenous injection of rivaroxaban at a dose of 10, 25 or 50 mg/kg. Thirty minutes later, mice were anesthetized with isoflurane and placed on a heated platform, and the body temperature of the mice was maintained at 37°C prior to the tail cut. The tails were immersed in 50 mL pre-warmed phosphate buffered saline (PBS) at 37°C for 2 minutes. A 3 mm tail cut was made and blood was collected into PBS for a 10 minute period. A quantitative assessment of the amount of bleeding was determined by hemoglobin content of the blood collected into PBS. Tubes were centrifuged to collect erythrocytes, resuspended in 5 mL lysis buffer (8.3 g/L NH₄Cl, 1.0 g/L KHCO₃, and 0.037 g/L EDTA), and the absorbance of the sample was measured at 575 nm. The absorbance values were converted to total blood loss (µL) using a standard curve. The administration of rivaroxaban resulted in a dose dependent increase in blood loss following a tail cut **(****Figure 11****).**

In this model, a dose of 50 mg/kg rivaroxaban resulted in an increase in blood loss following the tail transection. Mice were dosed with 50 mg/kg rivaroxaban and 30 minutes later 50 or 200 ug/kg of FXa^{I16L} was dosed intravenously at 37°C prior to the tail cut. Mice were then anesthetized with isoflurane and placed on a heated platform, and the body temperature of the mice was maintained at 37°C prior to the tail cut. The tails were immersed in 50 mLs pre-warmed phosphate buffered saline (PBS) at 37°C for 2 minutes. A 3 mm tail cut was made and blood was collected into PBS for a 10 minute period and the assessment of the amount of bleeding was determined by hemoglobin content as described. In this model, the administration of the hemostatic FXa^{I16L} variant decreased the excessive bleeding loss induced with rivaroxaban **(****Figure 11****).**

### Example 6 - FXa^{I16L} Counteracts Rivaroxaban in a Mouse Bleeding Model Demonstrated Using Intravital Microscopy

As visualized using intravital microscopy, rivaroxaban was demonstrated to inhibit thrombus formation in the microcirculation of the mouse cremaster muscle after laser-induced injury. Further administration of FXa^{I16L} could counteract the anti-coagulant effect of rivaroxaban in this system.

Using standard techniques, the cremaster muscle of mice was exposed and visualized using intravital microscopy. A vascular injury in the muscle was then induced using a laser. After injury, clot formation was visualized using different fluorescently labeled antibodies that specifically recognize fibrin and platelets. Clotting is indicated by the presence of fluorescent signal from both types of antibodies.

After laser injury, an untreated mouse rapidly formed a clot at the site of injury that was stable for several minutes **(****Figure 13A****)**. In the video frame, the clot is visible as the coincidence of fluorescent signal associated with antibodies against fibrin and platelets (light gray center region overlapping darker gray region). Administration of 1 mg/kg rivaroxaban to a mouse, however, delayed the accumulation of platelets at the injury site and eliminated any signs of fibrin **(****Figure 13B****)**. In the video frame, only a reduced extent of platelets can be seen as indicated by the dark gray region, which reflects presence of fluorescent signal associated with anti-platelet antibodies. By contrast, when a mouse was administered 1 mg/kg rivaroxaban followed by 0.5 mg/kg FXa^{I16L}, a clot rapidly formed at the injury site **(****Figure 13C****).** In the video frame, the clot is indicated by the characteristic pattern of fluorescent signal associated with antibodies against platelets and fibrin.

Unless otherwise defined herein, scientific and technical terms used in connection with the present disclosure shall have the meanings that are commonly understood by those of ordinary skill in the art. Further, unless otherwise required by context, singular terms shall include pluralities and plural terms shall include the singular. Generally, nomenclature used in connection with, and techniques of, cell and tissue culture, molecular biology, immunology, microbiology, genetics and protein and nucleic acid chemistry and hybridization described herein are those well known and commonly used in the art.

The methods and techniques of the present disclosure are generally performed according to conventional methods well known in the art and as described in various general and more specific references that are cited and discussed throughout the present specification unless otherwise indicated. See, e.g., Sambrook J. & Russell D.. Molecular Cloning: A Laboratory Manual, 3rd ed., Cold Spring Harbor Laboratory Press, Cold Spring Harbor, N.Y. (2000); Ausubel et al., Short Protocols in Molecular Biology: A Compendium of Methods from Current Protocols in Molecular Biology, Wiley, John & Sons, Inc. (2002); Harlow and Lane Using Antibodies: A Laboratory Manual, Cold Spring Harbor Laboratory Press, Cold Spring Harbor, N.Y. (1998); and Coligan et al., Short Protocols in Protein Science, Wiley, John & Sons, Inc. (2003), incorporated herein by reference. Enzymatic reactions and purification techniques are performed according to manufacturer's specifications, as commonly accomplished in the art or as described herein. The nomenclature used in connection with, and the laboratory procedures and techniques of, analytical chemistry, synthetic organic chemistry, and medicinal and pharmaceutical chemistry described herein are those well known and commonly used in the art.

Throughout this specification and claims, the word "comprise," or variations such as "comprises" or "comprising," will be understood to imply the inclusion of a stated integer or group of integers but not the exclusion of any other integer or group of integers.

## Claims

1. A Factor Xa variant comprising a substitution at the amino acid corresponding to position 235 in SEQ ID NO:1 selected from the group of amino acids consisting of: Thr or Leu, or a pharmaceutical composition thereof, for use in reducing or preventing bleeding in a subject being treated with a direct Factor Xa inhibitor.

2. The Factor Xa variant or pharmaceutical composition thereof for use according to claim 1, wherein the use effects the urgent reversal of acquired coagulopathy due to FXa inhibition therapy in a subject with acute major bleeding.

3. The Factor Xa variant or pharmaceutical composition thereof for use according to any of the previous claims, wherein there is a reduction in bleeding of at least 5%-10%, 10%-15%, 15%-20%, 20%-25%, 25%-30%, 30%-35%, 35%-40%, 40%-45%, 45%-50%, 50%-55%, 55%-60%, 60%-65%, 65%-70%, 70%-75%, 75%-80%, 80%-85%, 85%-90%, 90%-95%, or 95%-100%.

4. The Factor Xa variant or pharmaceutical composition thereof for use according to any of the previous claims, wherein the Factor Xa variant is administered before a planned surgery, after an injury or after a direct Factor Xa inhibitor overdose.

5. The Factor Xa variant or pharmaceutical composition thereof for use according to any of the previous, wherein Factor Xa variant is administered more than one time.

6. The Factor Xa variant or pharmaceutical composition thereof for use according to any of the previous claims, wherein at least one additional procoagulant is administered, optionally, wherein the procoagulant is selected from the group consisting of: a different Factor Xa variant, Factor IX, Factor Xla, Factor XIIa, Factor VIII, Factor VIIa, FEIBA and prothrombin complex concentrate (PCC).

7. The Factor Xa variant or pharmaceutical composition thereof for use according to any of the previous claims, wherein the plasma concentration of the direct FXa inhibitor is a supratherapeutic amount.

8. The FXa variant or pharmaceutical composition thereof for use according to any of the previous claims, wherein the Factor Xa variant is administered in a dose range of 0.0001 to 50 mg/kg, 0.001 to 50 mg/kg, 0.001 to 5 mg/kg, 0.001 to 0.5 mg/kg, 0.001 to 0.05 mg/kg, 0.01 to 5 mg/kg, or 0.01 to 0.5 mg/kg.

9. The FXa variant or pharmaceutical composition thereof for use according to any of the previous claims, wherein the Factor Xa variant is administered to achieve a serum concentration in a range of 0.0003 to 300 nM, 0.003 to 300 nM, 0.03 to 300 nM, 0.003 to 30 nM, 0.03 to 30 nM, or 0.3 to 3 nM.

10. The FXa variant or pharmaceutical composition thereof for use according to any of the previous claims, wherein the Factor Xa variant counters the effect of the direct FXa inhibitor at a plasma concentration that is at least 10-fold, at least 25-fold, at least 50-fold, at least 100-fold, at least 250-fold, at least 500-fold, at least 1,000-fold, at least 2,500-fold, at least 5,000-fold, or at least 10,000-fold lower than the plasma concentration of the direct FXa inhibitor.

11. The FXa variant or pharmaceutical composition thereof for use according to any of the previous claims, wherein the Factor Xa variant counters the effect of the direct FXa inhibitor at a plasma concentration that is calculated by multiplying the plasma concentration of the direct FXa inhibitor by a conversion factor ranging from 0.1 x 10⁻⁴ to 1000 x 10⁻⁴.

12. The FXa variant or pharmaceutical composition thereof for use according to any of the previous claims, wherein the direct FXa inhibitor is rivaroxaban or apixaban.

13. The Factor Xa variant or pharmaceutical composition thereof for use according to any of the previous claims, wherein the plasma concentration of rivaroxaban is at least 100 nM, 200 nM, 300 nM, 400 nM, 500 nM, 600 nM, 700 nM, or 800 nM.

14. The Factor Xa variant or pharmaceutical composition thereof for use according to any of the previous claims, wherein the plasma concentration of apixaban is at least 50 nM, 100 nM, 150 nM, 200nM, 250 nM, 300 nM, 350 nM, or 400 nM.

## Patentansprüche

1. Faktor-Xa-Variante, umfassend eine Substitution an der Aminosäure, die Position 235 in SEQ ID NO: 1 entspricht, ausgewählt aus der Gruppe von Aminosäuren, bestehend aus: Thr oder Leu, oder eine pharmazeutische Zusammensetzung davon zur Verwendung in der Reduktion oder Prävention von Blutung bei einem Subjekt, das mit einem Direkt-Faktor-Xa-Inhibitor behandelt wird.

2. Faktor-Xa-Variante oder pharmazeutische Zusammensetzung davon zur Verwendung gemäß Anspruch 1, wobei die Verwendung die dringende Umkehrung von erworbener Koagulopathie aufgrund der FXa-Inhibierungstherapie bei einem Patienten mit akuten schweren Blutungen bewirkt.

3. Faktor-Xa-Variante oder pharmazeutische Zusammensetzung davon zur Verwendung gemäß einem der vorangehenden Ansprüche, wobei eine Reduktion der Blutung um wenigstens 5%-10%, 10%-15%, 15%-20%, 20%-25%, 25-30%, 30%-35%, 35%-40%, 40%-45%, 45%-50%, 50%-55%, 55%-60%, 60%-65%, 65%-70%, 70%-75%, 75%-80%, 80%-85%, 85%-90%, 90-95% oder 95%-100% vorliegt.

4. Faktor-Xa-Variante oder pharmazeutische Zusammensetzung davon zur Verwendung gemäß einem der vorangehenden Ansprüche, wobei die Faktor-Xa-Variante vor einer geplanten Operation, nach einer Verletzung oder nach einer Direkt-Faktor-Xa-Inhibitor-Überdosis verabreicht wird.

5. Faktor-Xa-Variante oder pharmazeutische Zusammensetzung davon zur Verwendung gemäß einem der vorangehenden Ansprüche, wobei die Faktor-Xa-Variante mehr als einmal verabreicht wird.

6. Faktor-Xa-Variante oder pharmazeutische Zusammensetzung davon zur Verwendung gemäß einem der vorangehenden Ansprüche, wobei wenigstens ein zusätzliches Prokoagulationsmittel verabreicht wird, wobei gegebenenfalls das Prokoagulationsmittel ausgewählt ist aus der Gruppe, bestehend aus: einer anderen Faktor-Xa-Variante, Faktor IX, Faktor XIa, Faktor XIIa, Faktor VIII, Faktor VIIa, FEIBA und Prothrombin-Komplexkonzentrat (PCC, prothrombin complex concentrate).

7. Faktor-Xa-Variante oder pharmazeutische Zusammensetzung davon zur Verwendung gemäß einem der vorangehenden Ansprüche, wobei die Plasmakonzentration vom Direkt-FXa-Inhibitor eine supratherapeutische Menge beträgt.

8. Faktor-Xa-Variante oder pharmazeutische Zusammensetzung davon zur Verwendung gemäß einem der vorangehenden Ansprüche, wobei die Faktor-Xa-Variante in einem Dosisbereich von 0,0001 bis 50 mg/kg, 0,001 bis 50 mg/kg, 0,001 bis 5 mg/kg, 0,001 bis 0,5 mg/kg, 0,001 bis 0,05 mg/kg, 0,01 bis 5 mg/kg oder 0,01 bis 0,5 mg/kg verabreicht wird.

9. Faktor-Xa-Variante oder pharmazeutische Zusammensetzung davon zur Verwendung gemäß einem der vorangehenden Ansprüche, wobei die Faktor-Xa-Variante verabreicht wird, um eine Serumkonzentration in einem Bereich von 0,0003 bis 300 nM, 0,003 bis 300 nM, 0,03 bis 300 nM, 0,003 bis 30 nM, 0,03 bis 30 nM oder 0,3 bis 3 nM zu erreichen.

10. Faktor-Xa-Variante oder pharmazeutische Zusammensetzung davon zur Verwendung gemäß einem der vorangehenden Ansprüche, wobei die Faktor-Xa-Variante der Wirkung des Direkt-FXa-Inhibitors bei einer Plasmakonzentration, die wenigstens 10-fach, wenigstens 25-fach, wenigstens 50-fach, wenigstens 100-fach, wenigstens 250-fach, wenigstens 500-fach, wenigstens 1000-fach, wenigstens 2500-fach, wenigstens 5000-fach oder wenigstens 10000-fach niedriger als die Plasmakonzentration des Direkt-FXa-Inhibitors ist, entgegenwirkt.

11. Faktor-Xa-Variante oder pharmazeutische Zusammensetzung davon zur Verwendung gemäß einem der vorangehenden Ansprüche, wobei die Faktor-Xa-Variante der Wirkung des Direkt-FXa-Inhibitors bei einer Plasmakonzentration, die durch Multiplizierung der Plasmakonzentration des Direkt-FXa-Inhibitors mit einem Umrechnungsfaktor im Bereich von 0,1 x 10⁻⁴ bis 1000 x 10⁻⁴ berechnet wird, entgegenwirkt.

12. Faktor-Xa-Variante oder pharmazeutische Zusammensetzung davon zur Verwendung gemäß einem der vorangehenden Ansprüche, wobei der Direkt-FXa-Inhibitor Rivaroxaban oder Apixaban ist.

13. Faktor-Xa-Variante oder pharmazeutische Zusammensetzung davon zur Verwendung gemäß einem der vorangehenden Ansprüche, wobei die Plasmakonzentration von Rivaxoraban wenigstens 100 nM, 200 nM, 300 nM, 400 nM, 500 nM, 600 nM, 700 nM oder 800 nM beträgt.

14. Faktor-Xa-Variante oder pharmazeutische Zusammensetzung davon zur Verwendung gemäß einem der vorangehenden Ansprüche, wobei die Plasmakonzentration von Apixaban wenigstens 50 nM, 100 nM, 150 nM, 200 nM, 250 nM, 300 nM, 350 nM oder 400 nM beträgt.

## Revendications

1. Variant du facteur Xa comprenant une substitution au niveau de l'acide aminé correspondant à la position 235 dans SEQ ID NO : 1 sélectionné dans le groupe d'acides aminés constitué par : Thr ou Leu, ou une composition pharmaceutique de celui-ci, pour son utilisation dans la réduction ou la prévention d'une hémorragie chez un sujet traité par un inhibiteur direct du facteur Xa.

2. Variant du facteur Xa ou composition pharmaceutique de celui-ci pour son utilisation selon la revendication 1, dans lequel l'utilisation effectue l'inversion urgente d'une coagulopathie acquise en raison d'une thérapie d'inhibition du FXa chez un sujet avec une hémorragie majeure aiguë.

3. Variant du facteur Xa ou composition pharmaceutique de celui-ci pour son utilisation selon l'une quelconque des revendications précédentes, dans lequel il y a une réduction de l'hémorragie d'au moins 5 % à 10 %, 10 % à 15 %, 15 % à 20 %, 20 % à 25 %, 25 % à 30 %, 30 % à 35 %, 35 % à 40 %, 40 % à 45 %, 45 % à 50 %, 50 % à 55 %, 55 % à 60 %, 60 % à 65 %, 65 % à 70 %, 70 % à 75 %, 75 % à 80 %, 80 % à 85 %, 85 % à 90 %, 90 % à 95 %, ou 95 % à 100 %.

4. Variant du facteur Xa ou composition pharmaceutique pour son utilisation selon l'une quelconque des revendications précédentes dans lequel le variant du facteur Xa est administré avant une intervention chirurgicale prévue, après une blessure ou après une surdose d'inhibiteur direct du facteur Xa.

5. Variant du facteur Xa ou composition pharmaceutique de celui-ci pour son utilisation selon l'une quelconque des revendications précédentes, dans lequel le variant du facteur Xa est administré plus d'une fois.

6. Variant du facteur Xa ou composition pharmaceutique de celui-ci pour son utilisation selon l'une quelconque des revendications précédentes, dans lequel au moins un procoagulant supplémentaire est administré, optionnellement, dans lequel le procoagulant est sélectionné dans le groupe constitué par : un variant du facteur Xa différent, le facteur IX, le facteur XIa, le facteur XIIa, le facteur VIII, le facteur VIIa, FEIBA, et le concentré de complexe prothrombique (PCC).

7. Variant du facteur Xa ou composition pharmaceutique de celui-ci pour son utilisation selon l'une quelconque des revendications précédentes, dans lequel la concentration plasmatique en inhibiteur direct du FXa est une quantité suprathérapeutique.

8. Variant du FXa ou composition pharmaceutique de celui-ci pour son utilisation selon l'une quelconque des revendications précédentes, dans lequel le variant du facteur Xa est administré dans une plage de doses de 0,0001 à 50 mg/kg, de 0,001 à 50 mg/kg, de 0,001 à 5 mg/kg, de 0,001 à 0,5 mg/kg, de 0,001 à 0,05 mg/kg, de 0,01 à 5 mg/kg ou de 0,01 à 0,5 mg/kg.

9. Variant du FXa ou composition pharmaceutique de celui-ci pour son utilisation selon l'une quelconque des revendications précédentes, dans lequel le variant du facteur Xa est administré pour atteindre une concentration sérique dans une plage de 0,0003 à 300 nM, de 0,003 à 300 nM, de 0,03 à 300 nM, de 0,003 à 30 nM, de 0,03 à 30 nM ou de 0,3 à 3 nM.

10. Variant du FXa ou composition pharmaceutique de celui-ci pour son utilisation selon l'une quelconque des revendications précédentes, dans lequel le variant du facteur Xa contre l'effet de l'inhibiteur direct du Fxa à une concentration plasmatique qui est au moins 10 fois, au moins 25 fois, au moins 50 fois, au moins 100 fois, au moins 250 fois, au moins 500 fois, au moins 1000 fois, au moins 2500 fois, au moins 5000 fois, ou au moins 10 000 fois inférieure à la concentration plasmatique de l'inhibiteur direct du FXa.

11. Variant du FXa ou composition pharmaceutique de celui-ci pour son utilisation selon l'une quelconque des revendications précédentes, dans lequel le variant du facteur Xa contre l'effet de l'inhibiteur direct du FXa à une concentration plasmatique qui est calculée par multiplication de la concentration plasmatique de l'inhibiteur direct du FXa par un facteur de conversion dans la plage de 0,1 x 10⁻⁴ à 1000 x 10⁻⁴.

12. Variant du FXa ou composition pharmaceutique de celui-ci pour son utilisation selon l'une quelconque des revendications précédentes, dans lequel l'inhibiteur direct du FXa est le rivaroxaban ou l'apixaban.

13. Variant du facteur Xa ou composition pharmaceutique de celui-ci pour son utilisation selon l'une quelconque des revendications précédentes, dans lequel la concentration plasmatique en rivaroxaban est d'au moins 100 nM, 200 nM, 300 nM, 400 nM, 500 nM, 600 nM, 700 nM, ou 800 nM.

14. Variant du facteur Xa ou composition pharmaceutique de celui-ci pour son utilisation selon l'une quelconque des revendications précédentes, dans lequel la concentration plasmatique en apixaban est d'au moins 50 nM, 100 nM, 150 nM, 200nM, 250 nM, 300 nM, 350 nM, ou 400 nM.
